# EUROPEAN PATENT APPLICATION

(11) **EP 2 385 129 A1**
(43) Date of publication of application: **09.11.2011**
(21) Application number: 10161774.4
(22) Date of filing: 03.05.2010
(51) Int. Cl.: C12N 15/82

(54) **Enhanced methods for gene regulation in plants**

(71) Applicant: BASF Plant Science Company GmbH, 67056 Ludwigshafen (DE)
(72) Inventor: McMillan, John, Raleigh, NC 27612 (US); Dietz, Jon, Cary, NC 27519 (US); Deppong, Dave, Cary, NC 27511 (US); Xu, Nanfei, Cary, NC 27519 (US); Darnielle, Lalitree, Durham, NC 27707-1359 (US); McCaskill, Amy, Apex, NC 27502 (US); Ren, Peifeng, Cary, NC 27519 (US); Diener, Stephen, Raleigh, NC 27603 (US); Sarria-Millan, Rodrigo, Cary, NC 27519 (US)
(74) Representative: Popp, Andreas

(57) **Abstract**

The present invention is in the field of plant molecular biology and provides methods for regulating target gene expression in plants by suppression of the activity of endogenous microRNAs targeting said target genes.

## Description

Gene expression in plants is a highly controlled mechanism. Regulation takes place on all steps involved, for example the accessibility of the genomic DNA for the transcriptional machinery or regulation of stability of the messenger. In the last years it has been shown, that stability and accessibility of the messenger RNA is highly regulated by small interfering RNAs (siRNAs) such as, for example microRNAs, ta-siRNAs and others.

MicroRNAs have emerged as evolutionarily conserved, RNA-based regulators of gene expression in animals and plants. MicroRNAs (approx. 18 to 25 nt) arise from larger precursors, pre-miRNAs, with a stem loop structure that are transcribed from non-protein-coding genes.

Plant microRNAs known so far repress expression of a high number of genes which function in developmental processes, indicating that microRNA-based regulation is integral to pathways governing growth and development. Gene expression-repressing plant microRNAs usually contain near-perfect complementarity with target sites, which occur most commonly in protein-coding regions of mRNAs (Llave C et al. (2002) Science 297, 2053-2056; Rhoades MW et al. (2002) Cell 110, 513-520). As a result, in plants most gene expression-repressing plant microRNAs function to guide target RNA cleavage (Jones-Rhoades MW and Bartel DP (2004) Mol. Cell 14, 787-799; Kasschau KD et al. (2003) Dev. Cell 4, 205-217).

Various documents describe the use of microRNAs for downregulation of target gene expression by overexpression or induction of endogenous or recombinant microRNAs in plants. Ossowski et al (2008) are giving an overview on methods for gene silencing using artificial siRNAs such as microRNAs.

Overexpression of genes targeted by siRNAs, for example microRNAs is difficult to achieve, as the RNA derived from the target gene is rapidly inactivated by microRNA binding. One method to avoid such inactivation of target gene mRNA has been described in Tay et al (2008). The authors describe the introduction of mutations into the microRNA binding site of the messenger to avoid binding and thereby prevent degradation of the respective mRNA. This method is not always applicable, as the mutations introduced into the respective mRNA might affect functionality of the mRNA or the protein it is encoding. Therefore there is a need to overcome such limitations. Moreover, the mutated genes are generally not longer under control of the respective microRNA. Fine tuning, for example upregulation of the respective target gene in a specific tissue or at a specific developmental stage is not possible.

A method for increased expression of genes naturally targeted by microRNAs has been described in Esau et al (2006). The authors introduced chemically modified antisense oligonucleotides (ASO) into mice and showed increased mRNA levels of the respective target gene. This method can be applied to plant protoplasts. Introduction of such ASO in plants and maintenance of the effect over generations or even the lifetime of a plant is not feasible.

Recently, is has been shown in Arabidopsis that suppression of the activity of a specific microRNA leads to the induction or the increase of the mRNA of the respective target gene. Franco-Zorrilla et al (2007) showed that the mRNA of an endogenous gene expressed in Arabidopsis (IPS1 gene) is able to bind a specific microRNA that is not cleaving said mRNA. They show that thereby the microRNA is hindered to bind to the respective target gene leading to an increase of the mRNA of the gene targeted by the microRNA. In addition it has been shown, that by manipulating of the microRNA binding site in the IPS1 gene it was possible to bind other microRNAs thereby preventing these microRNAs from downregulation of their respective target genes. Such target mimicry genes are binding all microRNAs of a family that are sharing high sequence homology. Repression of a single member of a microRNA family is difficult as they often differ only in the sequence of the pre-microRNA. This regulation has yet only been demonstrated in Arabidopsis. There have been no reports of such gene regulation mechanisms in other plants.

Precise expression of recombinant genes in plants is a constant need in the art.

A multitude of methods for regulation of gene expression have been described. The invention at hand describes a further layer for regulation of target gene expression in plants. So far, no methods have been described using suppression of the activity of endogenous or recombinant small regulating RNAs (srRNAs), such as tasiRNA (trans activating siRNAs), nat-siRNAs (natural anti-sense siRNAs), hc-siRNA (heterochroamtic siRNAs), ca-siRNAs (cis-acting siRNAs), ImiRNAs (long miRNAs), IsiRNAs (long siRNAs) and easiRNA (epigenetically activated siRNA) or microRNAs to modulate the expression of the genes targeted by said small regulating RNAs in plants.

### Detailed description of the Invention

A first embodiment of the invention is directed to a method for modulating, compared to a respective wild-type plant or part thereof, the expression of at least one target gene in a plant or part thereof, wherein the method comprises the steps of
a) suppressing, compared to a respective wild-type plant or part thereof, the activity of at least one small regulating RNA (srRNA) molecule naturally targeting said target gene by
b) introducing into said plant or part thereof at least one recombinant nucleic acid molecule not occurring in a respective wild-type plant or part thereof, wherein at least a part of said recombinant nucleic acid molecule is complementary to at least a part of at least one srRNA molecule or at least one precursor of said at least one srRNA molecule comprising said srRNA molecule regulating expression of said target gene in said plant or part thereof.

SrRNA molecules are, for example microRNA molecules, ta-siRNA molecules (trans activating siRNAs), siRNA molecules, activating RNA (RNAa) molecules, nat-siRNA molecules (natural anti-sense siRNAs), hc-siRNA molecules (heterochroamtic siRNAs), ca-siRNA molecules (cis-acting siRNAs), ImiRNA molecules (long miRNAs), IsiRNA molecules (long siRNAs) and easiRNA molecules (epigenetically activated siRNA) and their respective precursors. Preferred srRNA molecules of the invention are microRNA molecules, ta-siRNA molecules and RNAa molecules and their respective precursors.

The term "introducing" is to be understood as genetic transformation of the respective plant or part thereof. This transformation may be transient transformation by means of for example particle bombardment, virus vectors, infiltration or the like. Transformation may also mean stable transformation, which comprises the step of introducing a recombinant nucleic acid molecule into the genome of a plant or part thereof, for example Agrobacterium mediated transformation. The skilled person is aware of other methods for transient or stable transformation of plants or parts thereof. In a preferred embodiment of the invention, the introduction of the recombinant nucleic acid molecule into a plant or part thereof is a stable transformation.

The recombinant nucleic acid molecule of the invention is in a preferred embodiment functionally linked to other regulatory nucleic acid molecules, for example a plant specific promoter and/or a terminator being heterologous to the recombinant nucleic acid molecule.

The skilled person is aware of the fact that srRNAs, mostly described for downregulation of their respective target gene, are also involved in increasing target gene expression. Hence, the embodiments of the invention can respectively be used to repress or increase target gene expression by suppressing, compared to a respective wild-type plant or part thereof, the activity of a srRNA molecule naturally targeting the target gene, depending on whether the srRNA molecule is increasing or repressing the expression of its respective target gene. All embodiments described herein read therefore as increasing or repressing target gene expression when for example the term "modulating" is used.

Increasing or repressing target gene expression in a plant or part thereof is to be understood as increasing or repressing target gene expression in a plant or part thereof compared to a wild-type or reference plant. Increase or repression may thereby be in a specific cell, cell layer, tissue or organ of the plant or may be in a specific developmental state of the plant such as senescence, germination or flowering or may be under specific conditions such as biotic or abiotic stress. The skilled person knows methods to measure expression of a target gene such as run-on experiments, quantitative RT-PCR, protein activity or protein quantification. The increase or decrease of the target gene expression is preferentially statistically significant, determined by for example the students T-test.

The person skilled in the art is aware of how to identify a srRNA molecule, for example a microRNA molecule that is targeting a specific target gene the expression of the latter is to be increased or repressed. Such methods are for example described in Cell (2009) 136, 669-687. After the identification of such srRNA molecule a precursor RNA molecule, for example a pre-miRNA molecule, comprising the srRNA sequence, for example the microRNA sequence may be identified. Such methods are for example described in Cell (2002) 110, 513-520.

The recombinant nucleic acid molecule not occurring in a respective wild-type plant or part thereof may for example be produced by identifying the sequence of a naturally occurring srRNA for example microRNA targeting a respective target gene. In a second step a nucleic acid molecule naturally suppressing activity of a natural srRNA is isolated. The nucleic acid molecule may for example suppress the activity of a srRNA by binding said srRNA molecule but preventing cleavage of the nucleic acid molecule. The binding may be facilitated by sequences comprised in said nucleic acid molecule that are at least partially inverse complement to the respective srRNA molecule. The sequence that is at least partially inverse complement to a natural srRNA molecule may in a further step be replaced by a sequence that is at least partially inverse complement to a srRNA molecule targeting the target gene. This recombinant nucleic acid molecule may further be functionally linked to plant regulatory nucleic acid molecule such as plant specific promoters, terminators and the like. The recombinant nucleic acid molecule may then be introduced into a plant or part thereof now suppressing the activity of the srRNA molecule identified in the first step of the method. Another embodiment of the method of the invention is to introduce into a plant or part thereof a recombinant nucleic acid molecule which comprises a sequence that is at least partially inverse complement to the precursor sequence of a srRNA molecule.

In a preferred embodiment of the method of the invention, the part of the recombinant nucleic acid molecule complementary to the precursor RNA molecule for example pre-miRNA molecules comprises 20 consecutive basepairs or more, for example 20 consecutive basepairs, or comprises 50 consecutive basepairs or more, for example 50 consecutive basepairs, preferably 100 consecutive complementary basepairs or more, for example 100 consecutive basepairs, 200 consecutive complementary basepairs or more, for example 200 consecutive basepairs, 300 consecutive complementary basepairs or more, for example 300 consecutive basepairs, more preferably 500 consecutive complementary basepairs or more, for example 500 consecutive basepairs of said precursor RNA molecule for example pre-miRNA molecule. The homology between the precursor RNA sequence for example pre-miRNA sequence and the consecutive complementary sequence of the respective recombinant nucleic acid molecule may for example be 80% or more, for example 80%, 85% or more, for example 85%, preferably 90% or more, for example 90%, 95% or more, for example 95%, more preferably 98% or more, for example 98%, even more preferably 99% or more, for example 99%. In a most preferred embodiment the homology between the precursor RNA sequence for example pre-miRNA sequence and the consecutive complementary sequence of the respective recombinant nucleic acid molecule is 100%.

In one embodiment of the invention, the recombinant nucleic acid molecule complementary to the precursor RNA molecule, for example the pre-miRNA molecule comprises sequences which are not complementary to the srRNA sequence for example the microRNA sequence comprised in said precursor RNA molecule, for example the pre-miRNA molecule.

The skilled person is aware, that in plants a multitude of srRNA molecules, for example microRNA molecules exist. Some of these srRNA molecules for example microRNA molecules have a 100% sequence identity or share a high degree of homology although derived from different precursor RNAs for example pre-miRNA molecules. By applying this embodiment of the invention the downregulation of a specific precursor RNA for example pre-miRNA molecules is achieved. This leads to the downregulation of one specific srRNA molecule for example microRNA molecule whereas the other members of the respective srRNA family for example microRNA family are unaffected. This allows a very precise regulation of a target gene.

In a further embodiment of the invention, the recombinant nucleic acid molecule complementary to the precursor RNA molecule, for example pre-miRNA molecule comprises sequences which are complementary to the srRNA sequence for example microRNA sequence comprised in said precursor RNA molecule for example pre-miRNA molecule.

The skilled person is aware, that in plants a multitude of srRNA molecules exist. Some of these srRNA molecules have a 100% sequence identity or share a high degree of homology although derived from different precursor RNAs. This has been shown for example for microRNAs derived from different pre-miRNAs (Cell (2002) 110, 513-520).

By applying this embodiment of the invention the repression of a family of srRNA molecules is achieved. This allows the downregulation of all srRNA molecules targeting the same target site of a specific target gene and allows an additional layer of regulation of this target gene.

The part of the recombinant nucleic acid molecule that is complementary to at least a part of the respective precursor RNA molecule comprising sequences complementary to the srRNA sequence comprised in said precursor RNA molecule may comprise at least the entire srRNA sequence comprised in said precursor RNA molecule but may comprise additional sequences adjacent to the srRNA sequence in the precursor RNA molecule comprising said srRNA sequence. The additional sequences may be adjacent to the 5', 3' or both sides of the srRNA sequence comprised in said precursor RNA molecule and may comprise for example 1 bp or more, 5 bp or more or 10 basepairs or more.

It may also be possible, that the part of the recombinant nucleic acid molecule that is complementary to at least a part of the respective precursor RNA molecule which is comprising sequences complementary to the srRNA sequence comprised in said precursor RNA molecule comprises sequences complementary to the entire srRNA sequence without any sequences adjacent to the srRNA sequence.

It may also be possible, that the part of the recombinant nucleic acid molecule that is complementary to at least a part of the respective precursor RNA molecule which is comprising sequences complementary to the srRNA sequence comprised in said precursor RNA molecule comprises sequences complementary to only a part of the srRNA sequence with or without any sequences adjacent to the srRNA sequence.

In one embodiment, the part of the recombinant nucleic acid molecule that is complementary to at least a part of the respective precursor RNA molecule which is comprising sequences complementary to the srRNA sequence comprised in said precursor RNA molecule may comprise for example 15 consecutive basepairs or more, for example 15, preferably 16 consecutive basepairs or more, for example 16, or 17 consecutive basepairs, 18 consecutive basepairs or 19 consecutive basepairs or more, more preferably 20 consecutive basepairs or 21 consecutive complementary basepairs or more, for example 21 of said srRNA sequence. The consecutive complementary basepairs comprised in the recombinant nucleic acid molecule may comprise 5 or less, for example 5, preferably 4 or less, for example 4, more preferably 3 or less, for example 3, 2 or one mismatch. In a most preferred embodiment the complementary consecutive basepairs comprise 3, 2 or 1 mismatch. In a preferred embodiment, these mismatches are consecutive.

In another embodiment, the consecutive complementary basepairs comprised in the recombinant nucleic acid molecule may be not completely consecutive but interrupted by additional basepairs of at least one insertion of 1 or more, for example 1, 2 or more, for example 2, 3 or more for example 3, 4 or more for example 4, 5 or more for example 5, 6 or more for example 6, 7 or more for example 7, 8 or more for example 8, 9 or more for example 9, 10 or more for example 10 additional basepairs not comprised in the precursor RNA molecule. In a preferred embodiment, the consecutive basepairs comprise one said insertion. In a preferred embodiment, the additional basepairs are consecutive and form a loop upon hybridization or basepairing to the respective part of the precursor RNA molecule or srRNA molecule. In a most preferred embodiment, the complementary basepairs comprise an insertion of 3 consecutive additional basepairs. The additional basepairs may be inserted at any position within the complementary basepairs comprised in the recombinant nucleic acid molecule. In a preferred embodiment the additional basepairs are inserted in the middle of the complementary basepairs.

In a most preferred embodiment the part of the recombinant nucleic acid molecule that is complementary to at least a part of the respective precursor RNA molecule which is comprising sequences complementary to the srRNA sequence comprised in said precursor RNA molecule is comprising 21 basepairs complementary to the srRNA molecule and this 21 basepairs complementary to the srRNA molecule are comprising an insertion of 3 consecutive additional basepairs between position 10 and 11 of said 21 basepairs complementary to the srRNA molecule.

The invention further provides a method for modulating, compared to a respective wild-type plant or part thereof, the expression of a target gene in a plant or part thereof wherein the recombinant nucleic acid molecule comprising a sequence complementary to a srRNA sequence for example microRNA sequence is selected from a nucleic acid molecule comprised in the group consisting of
a) a nucleic acid molecule represented by any of SEQ ID NOs: 1, 2, 7, 8, 9, 10, 11, 12 or 26 or
b) a nucleic acid molecule having at least 50, preferably 100, 150, 200 or 250 consecutive base pairs of a sequence described by any of SEQ ID NOs: 1, 2, 7, 8, 9, 10, 11, 12 or 26 or
c) a nucleic acid molecule having an identity of at least 70%, 75%, 80% or 85%, preferably 90%, 95%, 98% or 99%, most preferably 100% over a sequence of at least 95, 100, 110, 120, 130, 140 or 150 consecutive nucleic acid base pairs to a sequences described by any of SEQ ID NOs: 1, 2, 7, 8, 9, 10, 11, 12 or 26 or
d) a nucleic acid molecule hybridizing under medium stringent conditions, preferably high stringent condition, especially very high stringent conditions with a nucleic acid molecule of at least 50, 100, 150 or 200 consecutive base pairs of a nucleic acid molecule described by any of SEQ ID NOs: 1, 2, 7, 8, 9, 10, 11, 12 or 26.

It is a further embodiment of the invention, that the part of the recombinant nucleic acid molecule complementary to the respective precursor RNA, for example pre-miRNA is represented by position 347 bp - 370 bp of SEQ ID NO: 1, position 234 bp -- 257 bp of SEQ ID NO: 2, position 299 bp - 322 bp of SEQ ID NO: 7, position 233 bp - 256 bp of SEQ ID NO: 8, position 221 bp - 244 bp of SEQ ID NO: 9, position 184 bp - 207 bp of SEQ ID NO: 10, 149 bp-172 bp of SEQ ID NO: 11, position 49 bp-72 bp of SEQ ID NO: 12, and position 218 bp - 241 bp of SEQ ID NO: 26.

In a further embodiment of the invention SEQ ID NO: 7, 8, 9, 10, 11, 12 and/or 26 is modulating, increasing or repressing the expression of a target gene in a dicotyledonous plant, preferably leguminous plant, especially preferably in a plant of the genus Glycine, most preferably a Glycine max plant.

In a further embodiment of the invention SEQ ID NO: 1 is modulating, increasing or repressing the expression of a target gene in a monocotyledonous plant, preferably poacea plant, especially preferably in a plant of the genus Zea, most preferably a Zea mays plant.

In a further embodiment of the invention SEQ ID NO: 2 is modulating, increasing or repressing the expression of a target gene in a monocotyledonous plant, preferably poacea plant, especially preferably in a plant of the genus Oryca, most preferably a Oryza sativa plant.

The methods of the invention may be applied to any known srRNA molecule or srRNA precursor molecule. In a preferred embodiment, the methods are applied to any known microRNA molecule or pre-miRNA molecule. In a more preferred embodiment the sequence complementary to the respective precursor RNA sequence may be any of the sequences selected from the group of SEQ ID NO: 4512 to 16344 or a homolog or fragment or fragment of a homolog thereof.

A homolog thereof may be any sequence having at least 75%, 80%, 85%, 90%, 95%, 96%, 97%, 98%, 99% or 100% homology to any of the sequences selected from the group of SEQ ID NO: 4512 to 16344.

A fragment thereof may be any sequence comprising at least 10, 15, 16, 17, 18, 19, 20, 21, 50, 100, 150, 200 or 250 consecutive basepairs of the sequences selected from the group of SEQ ID NO: 4512 to 16344. In a preferred embodiment thereof, the fragment is consisting of at least 21 consecutive basepairs of the sequences selected from the group of SEQ ID NO: 4512 to 16344.

A fragment of a homolog may be any sequence having at least 75%, 80%, 85%, 90%, 95%, 96%, 97%, 98%, 99% or 100% homology and comprising at least 10, 15, 16, 17, 18, 19, 20, 21, 50, 100, 150, 200 or 250 consecutive basepairs of the sequences selected from the group of SEQ ID NO: 4512 to 16344. In a preferred embodiment thereof, a fragment of a homolog may be any sequence having at least 95% homology and is consisting of at least 21 consecutive basepairs of the sequences selected from the group of SEQ ID NO: 4512 to 16344.

In another preferred embodiment, the sequence complementary to the respective srRNA may be any of the sequences selected from the group of SEQ ID NO: 78 to 4511 or a homolog or fragment or fragment of a homolog thereof.

A homolog thereof may be any sequence having at least 75%, 80%, 85%, 90%, 95%, 96%, 97%, 98%, 99% or 100%homology to any of the sequences selected from the group of SEQ ID NO: 78 to 4511.

A fragment thereof may be any sequence comprising at least 10, 15, 16, 17, 18, 19, 20 or 21 consecutive basepairs of the sequences selected from the group of SEQ ID NO: 78-4511. In a preferred embodiment thereof, the fragment is consisting of at least 21 consecutive basepairs of the sequences selected from the group of SEQ ID NO: 78-4511.

A fragment of a homolog may be any sequence having at least 75%, 80%, 85%, 90%, 95%, 96%, 97%, 98%, 99% or 100% homology and comprising at least 10, 15, 16, 17, 18, 19, 20 or 21 consecutive basepairs of the sequences selected from the group of SEQ ID NO: 78 to 4511. In a preferred embodiment thereof, a fragment of a homolog may be any sequence having at least 95% homology and is consisting of at least 21 consecutive basepairs of the sequences selected from the group of SEQ ID NO: 78 to 4511.

It is a further preferred embodiment, that SEQ ID NO: 7, 8, 9, 10, 11, 12 and/or 26 comprises any of the sequences selected from the group of SEQ ID NO: 7168 to 16344 or a homolog or fragment or fragment of a homolog thereof.

A homolog thereof may be any sequence having at least 75%, 80%, 85%, 90%, 95%, 96%, 97%, 98%, 99% or 100% homology to any of the sequences selected from the group of SEQ ID NO: 7168 to 16344.

A fragment thereof may be any sequence comprising at least 10, 15, 16, 17, 18, 19, 20, 21, 50, 100, 150, 200 or 250 consecutive basepairs of the sequences selected from the group of SEQ ID NO: 7168 to 16344. In a preferred embodiment thereof, the fragment is consisting of at least 21 consecutive basepairs of the sequences selected from the group of SEQ ID NO: 7168 to 16344.

A fragment of a homolog may be any sequence having at least 75%, 80%, 85%, 90%, 95%, 96%, 97%, 98%, 99% or 100% homology and comprising at least 10, 15, 16, 17, 18, 19, 20, 21, 50, 100, 150, 200 or 250 consecutive basepairs of the sequences selected from the group of SEQ ID NO: 7168 to 16344. In a preferred embodiment thereof, a fragment of a homolog may be any sequence having at least 95% homology and is consisting of at least 21 consecutive basepairs of the sequences selected from the group of SEQ ID NO: 7168 to 16344.

It is a further preferred embodiment, that SEQ ID NO: 7, 8, 9, 10, 11, 12 and/or 26 comprises any of the sequences selected from the group of SEQ ID NO: 1404 to 4511 or a homolog or fragment or fragment of a homolog thereof.

A homolog thereof may be any sequence having at least 75%, 80%, 85%, 90%, 95%, 96%, 97%, 98%, 99% or 100% homology to any of the sequences selected from the group of SEQ ID NO: 1404 to 4511.

A fragment thereof may be any sequence comprising at least 10, 15, 16, 17, 18, 19, 20 or 21 consecutive basepairs of the sequences selected from the group of SEQ ID NO: 1404 to 4511. In a preferred embodiment thereof, the fragment is consisting of at least 21 consecutive basepairs of the sequences selected from the group of SEQ ID NO: 1404 to 4511.

A fragment of a homolog may be any sequence having at least 75%, 80%, 85%, 90%, 95%, 96%, 97%, 98%, 99% or 100% homology and comprising at least 10, 15, 16, 17, 18, 19, 20 or 21 consecutive basepairs of the sequences selected from the group of SEQ ID NO: 1404 to 4511. In a preferred embodiment thereof, a fragment of a homolog may be any sequence having at least 95% homology and is consisting of at least 21 consecutive basepairs of the sequences selected from the group of SEQ ID NO: 1404 to 4511.

It is a further preferred embodiment, that SEQ ID NO: 1 comprises any of the sequences selected from the group of SEQ ID NO: 4512 to 7167 or a homolog or fragment or fragment of a homolog thereof.

A homolog thereof may be any sequence having at least 75%, 80%, 85%, 90%, 95%, 96%, 97%, 98%, 99% or 100%homology to any of the sequences selected from the group of SEQ ID NO: 4512 to 7167.

A fragment thereof may be any sequence comprising at least 10, 15, 16, 17, 18, 19, 20, 21, 50, 100, 150, 200 or 250 consecutive basepairs of the sequences selected from the group of SEQ ID NO: 4512 to 7167. In a preferred embodiment thereof, the fragment is consisting of at least 21 consecutive basepairs of the sequences selected from the group of SEQ ID NO: 4512 to 7167.

A fragment of a homolog may be any sequence having at least 75%, 80%, 85%, 90%, 95%, 96%, 97%, 98%, 99% or 100%homology and comprising at least 10, 15, 16, 17, 18, 19, 20, 21, 50, 100, 150, 200 or 250 consecutive basepairs of the sequences selected from the group of SEQ ID NO: 4512 to 7167. In a preferred embodiment thereof, a fragment of a homolog may be any sequence having at least 95% homology and is consisting of at least 21 consecutive basepairs of the sequences selected from the group of SEQ ID NO: 4512 to 7167.

It is a further preferred embodiment, that SEQ ID NO: 1 comprises any of the sequences selected from the group of SEQ ID NO: 78 to 1403 or a homolog or fragment or fragment of a homolog thereof.

A homolog thereof may be any sequence having at least 75%, 80%, 85%, 90%, 95%, 96%, 97%, 98%, 99% or 100%homology to any of the sequences selected from the group of SEQ ID NO: 78 to 1403.

A fragment thereof may be any sequence comprising at least 10, 15, 16, 17, 18, 19, 20 or 21 consecutive basepairs of the sequences selected from the group of SEQ ID NO: 78 to 1403. In a preferred embodiment thereof, the fragment is consisting of at least 21 consecutive basepairs of the sequences selected from the group of SEQ ID NO: 78 to 1403.

A fragment of a homolog may be any sequence having at least 75%, 80%, 85%, 90%, 95%, 96%, 97%, 98%, 99% or 100%homology and comprising at least 10, 15, 16, 17, 18, 19, 20 or 21 consecutive basepairs of the sequences selected from the group of SEQ ID NO: 78 to 1403. In a preferred embodiment thereof, a fragment of a homolog may be any sequence having at least 95% homology and is consisting of at least 21 consecutive basepairs of the sequences selected from the group of SEQ ID NO: 78 to 1403.

The methods described above comprise the steps of
a) Identification of a srRNA sequence for example microRNA sequence targeting the target gene
b) Identification of the precursor RNA molecule for example the pre-miRNA precursor molecule comprising the respective srRNA sequence for example microRNA sequence
c) Producing the recombinant molecules, such as miRNA target mimics, miRNA trap or antisense transcript of pre-miRNA as described above and below in 'Examples'.
d) introducing the recombinant molecule into a plant or plant cell.

Methods to identify srRNA sequences are for example in silico prediction (Cell 110: 513-520) or deep sequencing (Nature Biotechnology 2007. 25:473-477). Methods to identify precursor RNA molecules are for example sequence analysis as described in Cell 2002; 110: 513-520.

For the production of the recombinant molecule of the invention any method known in the art may be applied. Such method may be restriction and ligation, recombination or synthesize of the respective recombinant molecule.

For the introduction of the recombinant molecule into a plant or plant cell various methods well known to a skilled person may be applied. For example particle bombardment, Agrobacterium mediated transformation, virus mediated transformation, electroporation and the like may be applied. The introduction into the plant or plant cell may lead to stable or transient transformation, preferably stable transformation.

A further embodiment of the present invention is a method for producing a plant or part thereof with, compared to a respective control plant or part thereof, modulated increased or repressed expression of one or more target gene comprising the steps of introducing into the plant or part thereof one or more recombinant expression constructs or vectors of the invention comprising a nucleic acid molecule as defined above.

Producing a plant as used herein comprises methods for stable transformation such as introducing a recombinant DNA construct into a plant or part thereof by means of Agrobacterium mediated transformation, protoplast transformation, particle bombardment or the like and optionally subsequent regeneration of a transgenic plant. It also comprises methods for transient transformation of a plant or part thereof such as viral infection or Agrobacterium infiltration. A skilled person is aware of further methods for stable and/or transient transformation of a plant or part thereof. Approaches such as breeding methods or protoplast fusion might also be employed for production of a plant of the invention and are covered herewith.

The method of the invention may be applied to any plant, for example gymnosperm or angiosperm, preferably angiosperm, for example dicotyledonous or monocotyledonous plants, preferably dicotyledonous plants. Preferred monocotyledonous plants are for example corn, wheat, rice, barley, sorghum, musa, sugarcane, miscanthus and brachypodium, especially preferred monocotyledonous plants are corn, wheat and rice. Preferred dicotyledonous plants are for example soy, rape seed, canola, linseed, cotton, potato, sugar beet, tagetes and *Arabidopsis,* especially preferred dicotyledonous plants are soy, rape seed, canola and potato

A further way to perform the methods of the invention may be to
a) provide an expression construct comprising one or more nucleic acid molecules comprising a sequence at least partially complementary to at least a part of a srRNA or a precursor RNA comprising a srRNA as defined above functionally linked to a plant specific promoter and
b) integrate said expression construct into the genome of said plant or part thereof and optionally
c) regenerate a plant or part thereof comprising said one or more expression construct from said transformed plant or part thereof.

The expression construct may be integrated into the genome of the respective plant with any method known in the art. The integration may be random using methods such as particle bombardment or Agrobacterium mediated transformation. In a preferred embodiment, the integration is via targeted integration for example by homologous recombination.

Further embodiments of the invention are the isolated nucleic acid molecules comprising, preferably consisting of the sequence selected from the group of any one of
a) a nucleic acid molecule represented by any of SEQ ID NO: 1, 2, 7, 8, 9, 10, 11, 12 or 26 or
b) a nucleic acid molecule having at least 50, preferably 100, 150, 200 or 250 consecutive base pairs of a sequence described by any of SEQ ID NO: 1, 2, 7, 8, 9, 10, 11, 12 or 26 or
c) a nucleic acid molecule having an identity of at least 70%, 75%, 80% or 85%, preferably 90% 95%, 98% or 99%, most preferably 100% over a sequence of at least 95, 100, 110, 120, 130, 140, 150, 200 or 250 consecutive nucleic acid base pairs to a sequences described by any of SEQ ID NOs: 1, 2, 7, 8, 9, 10, 11, 12 or 26 or
d) a nucleic acid molecule hybridizing under medium stringency conditions, preferably high stringency condition, especially very high stringency conditions with a nucleic acid molecule of at least 50, 100, 150 or 200 consecutive base pairs of a nucleic acid molecule described by any of SEQ ID NOs: 1, 2, 7, 8, 9, 10, 11, 12 or 26.

A nucleic acid construct for expression in plants comprising a recombinant nucleic acid molecule as defined above functionally linked to a plant specific promoter is also an embodiment of the invention.

A skilled person is aware of various methods for functionally linking two or more nucleic acids molecules. Such methods may encompass restriction/ligation, ligase independent cloning, recombineering, recombination or synthesis. Other methods may be employed to functionally link two or more nucleic acids molecules.

A vector comprising a nucleic acid construct as defined above is a further embodiment of the invention.

A microorganism able to transfer nucleic acids to a plant or part of a plant wherein said microorganism is comprising a recombinant nucleic acid construct as defined above or a vector as defined above, wherein said recombinant nucleic acid molecule confers upon transfer of said recombinant nucleic acid construct into a plant or plant cell an increase or decrease of expression of a target gene in said plant or part of a plant compared to a respective plant or part of a plant not comprising said recombinant nucleic acid molecule is a further embodiment of the invention.

A plant cell comprising a recombinant nucleic acid construct as defined above or a vector as defined above, wherein said recombinant nucleic acid molecule confers an increase or decrease of expression of a target gene in said plant cell compared to a respective plant cell not comprising said recombinant nucleic acid molecule is also an embodiment of the invention.

A plant or part thereof comprising a recombinant nucleic acid construct of the invention or a vector of the invention, wherein said recombinant nucleic acid molecule confers an increase or decrease of expression of a target gene in said plant or part thereof compared to a respective plant or part thereof not comprising said recombinant nucleic acid molecule is additionally provided with this invention.

A further embodiment of the invention is any method comprising a nucleic acid construct of the invention, a plant of the invention and/or a plant cell of the invention.

Any method for production of a nucleic acid construct of the invention, a vector of the invention, a plant and/or a plant cell of the invention are further embodiments of the invention.

Any use of an expression construct of the invention or a vector of the invention for increasing or repressing compared to a wild-type plant or part thereof the expression of a target gene in a plant or part thereof is also part of the invention.

### DEFINITIONS

Abbreviations: GFP - green fluorescence protein, GUS - beta-Glucuronidase, BAP - 6-benzylaminopurine; 2,4-D - 2,4-dichlorophenoxyacetic acid; MS - Murashige and Skoog medium; NAA - 1-naphtaleneacetic acid; MES, 2-(N-morpholino-ethanesulfonic acid, IAA indole acetic acid; Kan: Kanamycin sulfate; GA3 - Gibberellic acid; Timentin™: ticarcillin disodium / clavulanate potassium.

It is to be understood that this invention is not limited to the particular methodology or protocols. It is also to be understood that the terminology used herein is for the purpose of describing particular embodiments only, and is not intended to limit the scope of the present invention which will be limited only by the appended claims. It must be noted that as used herein and in the appended claims, the singular forms "a," "and," and "the" include plural reference unless the context clearly dictates otherwise. Thus, for example, reference to "a vector" is a reference to one or more vectors and includes equivalents thereof known to those skilled in the art, and so forth. The term "about" is used herein to mean approximately, roughly, around, or in the region of. When the term "about" is used in conjunction with a numerical range, it modifies that range by extending the boundaries above and below the numerical values set forth. In general, the term "about" is used herein to modify a numerical value above and below the stated value by a variance of 20 percent, preferably 10 percent up or down (higher or lower). As used herein, the word "or" means any one member of a particular list and also includes any combination of members of that list. The words "comprise," "comprising," "include," "including," and "includes" when used in this specification and in the following claims are intended to specify the presence of one or more stated features, integers, components, or steps, but they do not preclude the presence or addition of one or more other features, integers, components, steps, or groups thereof. For clarity, certain terms used in the specification are defined and used as follows:

Antiparallel: "Antiparallel" refers herein to two nucleotide sequences paired through hydrogen bonds between complementary base residues with phosphodiester bonds running in the 5'-3' direction in one nucleotide sequence and in the 3'-5' direction in the other nucleotide sequence.

Antisense: The term "antisense" refers to a nucleotide sequence that is inverted relative to its normal orientation for transcription or function and so expresses an RNA transcript that is complementary to a target gene mRNA molecule expressed within the host cell (e.g., it can hybridize to the target gene mRNA molecule or single stranded genomic DNA through Watson-Crick base pairing) or that is complementary to a target DNA molecule such as, for example genomic DNA present in the host cell.

Coding region: As used herein the term "coding region" when used in reference to a structural gene refers to the nucleotide sequences which encode the amino acids found in the nascent polypeptide as a result of translation of a mRNA molecule. The coding region is bounded, in eukaryotes, on the 5'-side by the nucleotide triplet "ATG" which encodes the initiator methionine and on the 3'-side by one of the three triplets which specify stop codons (i.e., TAA, TAG, TGA). In addition to containing introns, genomic forms of a gene may also include sequences located on both the 5'- and 3'-end of the sequences which are present on the RNA transcript. These sequences are referred to as "flanking" sequences or regions (these flanking sequences are located 5' or 3' to the non-translated sequences present on the mRNA transcript). The 5'-flanking region may contain regulatory sequences such as promoters and enhancers which control or influence the transcription of the gene. The 3'-flanking region may contain sequences which direct the termination of transcription, post-transcriptional cleavage and polyadenylation.

Complementary: "Complementary" or "complementarity" refers to two nucleotide sequences which comprise antiparallel nucleotide sequences capable of pairing with one another (by the base-pairing rules) upon formation of hydrogen bonds between the complementary base residues in the antiparallel nucleotide sequences. For example, the sequence 5'-AGT-3' is complementary to the sequence 5'-ACT-3'. Complementarity can be "partial" or "total." "Partial" complementarity is where one or more nucleic acid bases are not matched according to the base pairing rules. "Total" or "complete" complementarity between nucleic acid molecules is where each and every nucleic acid base is matched with another base under the base pairing rules. The degree of complementarity between nucleic acid molecule strands has significant effects on the efficiency and strength of hybridization between nucleic acid molecule strands. A "complement" of a nucleic acid sequence as used herein refers to a nucleotide sequence whose nucleic acid molecules show total complementarity to the nucleic acid molecules of the nucleic acid sequence.

Double-stranded RNA: A "double-stranded RNA" molecule or "dsRNA" molecule comprises a sense RNA fragment of a nucleotide sequence and an antisense RNA fragment of the nucleotide sequence, which both comprise nucleotide sequences complementary to one another, thereby allowing the sense and antisense RNA fragments to pair and form a double-stranded RNA molecule.

Endogenous: An "endogenous" nucleotide sequence refers to a nucleotide sequence, which is present in the genome of the untransformed plant cell.

Enhanced expression: "enhance" or "increase" the expression of a nucleic acid molecule in a plant cell are used equivalently herein and mean that the level of expression of the nucleic acid molecule in a plant, part of a plant or plant cell after applying a method of the present invention is higher than its expression in the plant, part of the plant or plant cell before applying the method, or compared to a reference plant lacking a recombinant nucleic acid molecule of the invention. For example, the reference plant is comprising the same construct which is only lacking the respective part complementary to at least a part of the precursor molecule comprising the srRNA sequence. The term "enhanced" or "increased" as used herein are synonymous and means herein higher, preferably significantly higher expression of the nucleic acid molecule to be expressed. As used herein, an "enhancement" or "increase" of the level of an agent such as a protein, mRNA or RNA means that the level is increased relative to a substantially identical plant, part of a plant or plant cell grown under substantially identical conditions, lacking a recombinant nucleic acid molecule of the invention, for example lacking the respective part complementary to at least a part of the precursor molecule comprising the srRNA sequence, the recombinant construct or recombinant vector of the invention. As used herein, "enhancement" or "increase" of the level of an agent, such as for example a preRNA, mRNA, rRNA, tRNA, snoRNA, snRNA expressed by the target gene and/or of the protein product encoded by it, means that the level is increased 50% or more, for example 100% or more, preferably 200% or more, more preferably 5 fold or more, even more preferably 10 fold or more, most preferably 20 fold or more for example 50 fold relative to a cell or organism lacking a recombinant nucleic acid molecule of the invention. The enhancement or increase can be determined by methods with which the skilled worker is familiar. Thus, the enhancement or increase of the nucleic acid or protein quantity can be determined for example by an immunological detection of the protein. Moreover, techniques such as protein assay, fluorescence, Northern hybridization, nuclease protection assay, reverse transcription (quantitative RT-PCR), ELISA (enzyme-linked immunosorbent assay), Western blotting, radioimmunoassay (RIA) or other immunoassays and fluorescence-activated cell analysis (FACS) can be employed to measure a specific protein or RNA in a plant or plant cell. Depending on the type of the induced protein product, its activity or the effect on the phenotype of the organism or the cell may also be determined. Methods for determining the protein quantity are known to the skilled worker. Examples, which may be mentioned, are: the micro-Biuret method (Goa J (1953) Scand J Clin Lab Invest 5:218-222), the Folin-Ciocalteau method (Lowry OH et al. (1951) J Biol Chem 193:265-275) or measuring the absorption of CBB G-250 (Bradford MM (1976) Analyt Biochem 72:248-254). As one example for quantifying the activity of a protein, the detection of luciferase activity is described in the Examples below.

Expression: "Expression" refers to the biosynthesis of a gene product, preferably to the transcription and/or translation of a nucleotide sequence, for example an endogenous gene or a heterologous gene, in a cell. For example, in the case of a structural gene, expression involves transcription of the structural gene into mRNA and - optionally - the subsequent translation of mRNA into one or more polypeptides. In other cases, expression may refer only to the transcription of the DNA harboring an RNA molecule. Expression may also refer to the change of the steady state level of the respective RNA in a plant or part thereof for example due to change of the stability of the respective RNA.

Expression construct: "Expression construct" as used herein mean a DNA sequence capable of directing expression of a particular nucleotide sequence in an appropriate part of a plant or plant cell, comprising a promoter functional in said part of a plant or plant cell into which it will be introduced, operatively linked to the nucleotide sequence of interest which is - optionally - operatively linked to termination signals. If translation is required, it also typically comprises sequences required for proper translation of the nucleotide sequence. The coding region may code for a protein of interest but may also code for a functional RNA of interest, for example RNAa, siRNA, snoRNA, snRNA, microRNA, ta-siRNA or any other noncoding regulatory RNA, in the sense or antisense direction. The expression construct comprising the nucleotide sequence of interest may be chimeric, meaning that one or more of its components is heterologous with respect to one or more of its other components. The expression construct may also be one, which is naturally occurring but has been obtained in a recombinant form useful for heterologous expression. Typically, however, the expression construct is heterologous with respect to the host, i.e., the particular DNA sequence of the expression construct does not occur naturally in the host cell and must have been introduced into the host cell or an ancestor of the host cell by a transformation event. The expression of the nucleotide sequence in the expression construct may be under the control of a constitutive promoter or of an inducible promoter, which initiates transcription only when the host cell is exposed to some particular external stimulus. In the case of a plant, the promoter can also be specific to a particular tissue or organ or stage of development.

Foreign: The term "foreign" refers to any nucleic acid molecule (e.g., gene sequence) which is introduced into the genome of a cell by experimental manipulations and may include sequences found in that cell so long as the introduced sequence contains some modification (e.g., a point mutation, the presence of a selectable marker gene, etc.) and is therefore distinct relative to the naturally-occurring sequence.

Functional linkage: The term "functional linkage" or "functionally linked" is to be understood as meaning, for example, the sequential arrangement of a regulatory element (e.g. a promoter) with a nucleic acid sequence to be expressed and, if appropriate, further regulatory elements (such as e.g., a terminator or an enhancer) in such a way that each of the regulatory elements can fulfill its intended function to allow, modify, facilitate or otherwise influence expression of said nucleic acid sequence. As a synonym the wording "operable linkage" or "operably linked" may be used. The expression may result depending on the arrangement of the nucleic acid sequences in relation to sense or antisense RNA. To this end, direct linkage in the chemical sense is not necessarily required. Genetic control sequences such as, for example, enhancer sequences, can also exert their function on the target sequence from positions which are further away, or indeed from other DNA molecules. Preferred arrangements are those in which the nucleic acid sequence to be expressed recombinantly is positioned behind the sequence acting as promoter, so that the two sequences are linked covalently to each other. The distance between the promoter sequence and the nucleic acid sequence to be expressed recombinantly is preferably less than 200 base pairs, especially preferably less than 100 base pairs, very especially preferably less than 50 base pairs. In a preferred embodiment, the nucleic acid sequence to be transcribed is located behind the promoter in such a way that the transcription start is identical with the desired beginning of the chimeric RNA of the invention. Functional linkage, and an expression construct, can be generated by means of customary recombination and cloning techniques as described (e.g., in Maniatis T, Fritsch EF and Sambrook J (1989) Molecular Cloning: A Laboratory Manual, 2nd Ed., Cold Spring Harbor Laboratory, Cold Spring Harbor (NY); Silhavy et al.

(1984) Experiments with Gene Fusions, Cold Spring Harbor Laboratory, Cold Spring Harbor (NY); Ausubel et al. (1987) Current Protocols in Molecular Biology, Greene Publishing Assoc. and Wiley Interscience; Gelvin et al. (Eds) (1990) Plant Molecular Biology Manual; Kluwer Academic Publisher, Dordrecht, The Netherlands). However, further sequences, which, for example, act as a linker with specific cleavage sites for restriction enzymes, or as a signal peptide, may also be positioned between the two sequences. The insertion of sequences may also lead to the expression of fusion proteins. Preferably, the expression construct, consisting of a linkage of a regulatory region for example a promoter and nucleic acid sequence to be expressed, can exist in a vector-integrated form and be inserted into a plant genome, for example by transformation.

Gene: The term "gene" refers to a region operably joined to appropriate regulatory sequences capable of regulating the expression of the gene product (e.g., a polypeptide or a functional RNA) in some manner. A gene includes untranslated regulatory regions of DNA (e.g., promoters, enhancers, repressors, etc.) preceding (up-stream) and following (downstream) the coding region (open reading frame, ORF) as well as, where applicable, intervening sequences (i.e., introns) between individual coding regions (i.e., exons). The term "structural gene" as used herein is intended to mean a DNA sequence that is transcribed into mRNA which is then translated into a sequence of amino acids characteristic of a specific polypeptide.

Genome and genomic DNA: The terms "genome" or "genomic DNA" is referring to the heritable genetic information of a host organism. Said genomic DNA comprises the DNA of the nucleus (also referred to as chromosomal DNA) but also the DNA of the plastids (e.g., chloroplasts) and other cellular organelles (e.g., mitochondria). Preferably the terms genome or genomic DNA is referring to the chromosomal DNA of the nucleus.

Heterologous: The term "heterologous" with respect to a nucleic acid molecule or DNA refers to a nucleic acid molecule which is operably linked to, or is manipulated to become operably linked to, a second nucleic acid molecule to which it is not operably linked in nature, or to which it is operably linked at a different location in nature. A heterologous expression construct comprising a nucleic acid molecule and one or more regulatory nucleic acid molecule (such as a promoter or a transcription termination signal) linked thereto for example is a constructs originating by experimental manipulations in which either a) said nucleic acid molecule, or b) said regulatory nucleic acid molecule or c) both (i.e. (a) and (b)) is not located in its natural (native) genetic environment or has been modified by experimental manipulations, an example of a modification being a substitution, addition, deletion, inversion or insertion of one or more nucleotide residues. Natural genetic environment refers to the natural chromosomal locus in the organism of origin, or to the presence in a genomic library. In the case of a genomic library, the natural genetic environment of the sequence of the nucleic acid molecule is preferably retained, at least in part. The environment flanks the nucleic acid sequence at least at one side and has a sequence of at least 50 bp, preferably at least 500 bp, especially preferably at least 1,000 bp, very especially preferably at least 5,000 bp, in length. A naturally occurring expression construct - for example the naturally occurring combination of a promoter with the corresponding gene - becomes a transgenic expression construct when it is modified by non-natural, synthetic "artificial" methods such as, for example, mutagenization. Such methods have been described (US 5,565,350; WO 00/15815). For example a protein encoding nucleic acid molecule operably linked to a promoter, which is not the native promoter of this molecule, is considered to be heterologous with respect to the promoter. Preferably, heterologous DNA is not endogenous to or not naturally associated with the cell into which it is introduced, but has been obtained from another cell or has been synthesized. Heterologous DNA also includes an endogenous DNA sequence, which contains some modification, non-naturally occurring, multiple copies of an endogenous DNA sequence, or a DNA sequence which is not naturally associated with another DNA sequence physically linked thereto. Generally, although not necessarily, heterologous DNA encodes RNA or proteins that are not normally produced by the cell into which it is expressed.

Hybridization: The term "hybridization" as used herein includes "any process by which a strand of nucleic acid molecule joins with a complementary strand through base pairing." (J. Coombs (1994) Dictionary of Biotechnology, Stockton Press, New York). Hybridization and the strength of hybridization (i.e., the strength of the association between the nucleic acid molecules) is impacted by such factors as the degree of complementarity between the nucleic acid molecules, stringency of the conditions involved, the Tm of the formed hybrid, and the G:C ratio within the nucleic acid molecules. As used herein, the term "Tm" is used in reference to the "melting temperature." The melting temperature is the temperature at which a population of double-stranded nucleic acid molecules becomes half dissociated into single strands. The equation for calculating the Tm of nucleic acid molecules is well known in the art. As indicated by standard references, a simple estimate of the Tm value may be calculated by the equation: Tm=81.5+0.41(% G+C), when a nucleic acid molecule is in aqueous solution at 1 M NaCl [see e.g., Anderson and Young, Quantitative Filter Hybridization, in Nucleic Acid Hybridization (1985)]. Other references include more sophisticated computations, which take structural as well as sequence characteristics into account for the calculation of Tm. Stringent conditions, are known to those skilled in the art and can be found in Current Protocols in Molecular Biology, John Wiley & Sons, N.Y. (1989), 6.3.1-6.3.6.

Medium stringency conditions when used in reference to nucleic acid hybridization comprise conditions equivalent to binding or hybridization at 68°C in a solution consisting of 5x SSPE (43.8 g/L NaCl, 6.9 g/L NaH2PO4.H2O and 1.85 g/L EDTA, pH adjusted to 7.4 with NaOH), 1 % SDS, 5x Denhardt's reagent [50x Denhardt's contains the following per 500 mL 5 g Ficoll (Type 400, Pharmacia), 5 g BSA (Fraction V; Sigma)] and 100 µg/mL denatured salmon sperm DNA followed by washing (preferably for one times 15 minutes, more preferably two times 15 minutes, more preferably three time 15 minutes) in a solution comprising 1xSSC (1× SSC is 0.15 M NaCl plus 0.015 M sodium citrate) and 0.1 % SDS at room temperature or-preferably 37°C - when a DNA probe of preferably about 100 to about 500 nucleotides in length is employed.

Normal stringency conditions when used in reference to nucleic acid hybridization comprise conditions equivalent to binding or hybridization at 68°C in a solution consisting of 5x SSPE (43.8 g/L NaCl, 6.9 g/L NaH2PO4.H2O and 1.85 g/L EDTA, pH adjusted to 7.4 with NaOH), 1 % SDS, 5x Denhardt's reagent [50x Denhardt's contains the following per 500 mL 5 g Ficoll (Type 400, Pharmacia), 5 g BSA (Fraction V; Sigma)] and 100 µg/mL denatured salmon sperm DNA followed by washing (preferably for one times 15 minutes, more preferably two times 15 minutes, more preferably three time 15 minutes) in a solution comprising 0.1xSSC (1 ×SSC is 0.15 M NaCl plus 0.015 M sodium citrate) and 1% SDS at room temperature or-preferably 37°C - when a DNA probe of preferably about 100 to about 500 nucleotides in length is employed.

High stringency conditions when used in reference to nucleic acid hybridization comprise conditions equivalent to binding or hybridization at 68°C in a solution consisting of 5x SSPE (43.8 g/L NaCl, 6.9 g/L NaH2PO4.H2O and 1.85 g/L EDTA, pH adjusted to 7.4 with NaOH), 1 % SDS, 5x Denhardt's reagent [50x Denhardt's contains the following per 500 mL 5 g Ficoll (Type 400, Pharmacia), 5 g BSA (Fraction V; Sigma)] and 100 µg/mL denatured salmon sperm DNA followed by washing (preferably for one times 15 minutes, more preferably two times 15 minutes, more preferably three time 15 minutes) in a solution comprising 0.1xSSC (1 × SSC is 0.15 M NaCl plus 0.015 M sodium citrate) and 1% SDS at 50°C - when a DNA probe of preferably about 100 to about 500 nucleotides in length is employed.

Very high stringency conditions when used in reference to nucleic acid hybridization comprise conditions equivalent to binding or hybridization at 68°C in a solution consisting of 5x SSPE, 1% SDS, 5x Denhardt's reagent and 100 µg/mL denatured salmon sperm DNA followed by washing (preferably for one times 15 minutes, more preferably two times 15 minutes, more preferably three time 15 minutes) in a solution comprising 0.1 x SSC, and 1% SDS at 68°C, when a probe of preferably about 100 to about 500 nucleotides in length is employed.

"Identity": "Identity" when used in respect to the comparison of two or more nucleic acid or amino acid molecules means that the sequences of said molecules share a certain degree of sequence similarity, the sequences being partially identical.

To determine the percentage identity (homology is herein used interchangeably) of two amino acid sequences or of two nucleic acid molecules, the sequences are written one underneath the other for an optimal comparison (for example gaps may be inserted into the sequence of a protein or of a nucleic acid in order to generate an optimal alignment with the other protein or the other nucleic acid).

The amino acid residues or nucleic acid molecules at the corresponding amino acid positions or nucleotide positions are then compared. If a position in one sequence is occupied by the same amino acid residue or the same nucleic acid molecule as the corresponding position in the other sequence, the molecules are homologous at this position (i.e. amino acid or nucleic acid "homology" as used in the present context corresponds to amino acid or nucleic acid "identity". The percentage identity between the two sequences is a function of the number of identical positions shared by the sequences (i.e. % homology = number of identical positions/total number of positions x 100). The terms "homology" and "identity" are thus to be considered as synonyms.

For the determination of the percentage identity of two or more amino acids or of two or more nucleotide sequences several computer software programs have been developed. The identity of two or more sequences can be calculated with for example the software fasta, which presently has been used in the version fasta 3 (W. R. Pearson and D. J. Lipman, PNAS 85, 2444(1988); W. R. Pearson, Methods in Enzymology 183, 63 (1990); W. R. Pearson and D. J. Lipman, PNAS 85, 2444 (1988); W. R. Pearson, Enzymology 183, 63 (1990)). Another useful program for the calculation of identities of different sequences is the standard blast program, which is included in the Biomax pedant software (Biomax, Munich, Federal Republic of Germany). This leads unfortunately sometimes to suboptimal results since blast does not always include complete sequences of the subject and the query. Nevertheless as this program is very efficient it can be used for the comparison of a huge number of sequences. The following settings are typically used for such a comparisons of sequences:

-p Program Name [String]; -d Database [String]; default = nr; -i Query File [File In]; default = stdin; -e Expectation value (E) [Real]; default = 10.0; -m alignment view options: 0 = pairwise; 1 = query-anchored showing identities; 2 = query-anchored no identities; 3 = flat query-anchored, show identities; 4 = flat query-anchored, no identities; 5 = query-anchored no identities and blunt ends; 6 = flat query-anchored, no identities and blunt ends; 7 = XML Blast output; 8 = tabular; 9 tabular with comment lines [Integer]; default = 0; -o BLAST report Output File [File Out] Optional; default = stdout; -F Filter query sequence (DUST with blastn, SEG with others) [String]; default = T; -G Cost to open a gap (zero invokes default behavior) [Integer]; default = 0; -E Cost to extend a gap (zero invokes default behavior) [Integer]; default = 0; -X X dropoff value for gapped alignment (in bits) (zero invokes default behavior); blastn 30, megablast 20, tblastx 0, all others 15 [Integer]; default = 0; -I Show GI's in deflines [T/F]; default = F; -q Penalty for a nucleotide mismatch (blastn only) [Integer]; default = -3; -r Reward for a nucleotide match (blastn only) [Integer]; default = 1; -v Number of database sequences to show one-line descriptions for (V) [Integer]; default = 500; -b Number of database sequence to show alignments for (B) [Integer]; default = 250; -f Threshold for extending hits, default if zero; blastp 11, blastn 0, blastx 12, tblastn 13; tblastx 13, megablast 0 [Integer]; default = 0; -g Perfom gapped alignment (not available with tblastx) [T/F]; default = T; -Q Query Genetic code to use [Integer]; default = 1; -D DB Genetic code (for tblast[nx] only) [Integer]; default = 1; -a Number of processors to use [Integer]; default = 1; -O SeqAlign file [File Out] Optional; -J Believe the query defline [T/F]; default = F; -M Matrix [String]; default = BLOSUM62; -W Word size, default if zero (blastn 11, megablast 28, all others 3) [Integer]; default = 0; -z Effective length of the database (use zero for the real size) [Real]; default = 0; -K Number of best hits from a region to keep (off by default, if used a value of 100 is recommended) [Integer]; default = 0; -P 0 for multiple hit, 1 for single hit [Integer]; default = 0; -Y Effective length of the search space (use zero for the real size) [Real]; default = 0; -S Query strands to search against database (for blast[nx], and tblastx); 3 is both, 1 is top, 2 is bottom [Integer]; default = 3; -T Produce HTML output [T/F]; default = F; -I Restrict search of database to list of GI's [String] Optional; -U Use lower case filtering of FASTA sequence [T/F] Optional; default = F; -y X dropoff value for ungapped extensions in bits (0.0 invokes default behavior); blastn 20, megablast 10, all others 7 [Real]; default = 0.0; -Z X dropoff value for final gapped alignment in bits (0.0 invokes default behavior); blastn/megablast 50, tblastx 0, all others 25 [Integer]; default = 0; -R PSI-TBLASTN checkpoint file [File In] Optional; -n MegaBlast search [T/F]; default = F; -L Location on query sequence [String] Optional; -A Multiple Hits window size, default if zero (blastn/megablast 0, all others 40 [Integer]; default = 0; -w Frame shift penalty (OOF algorithm for blastx) [Integer]; default = 0; -t Length of the largest intron allowed in tblastn for linking HSPs (0 disables linking) [Integer]; default = 0.

Results of high quality are reached by using the algorithm of Needleman and Wunsch or Smith and Waterman. Therefore programs based on said algorithms are preferred. Advantageously the comparisons of sequences can be done with the program PileUp (J. Mol. Evolution., 25, 351 (1987), Higgins et al., CABIOS 5, 151 (1989)) or preferably with the programs "Gap" and "Needle", which are both based on the algorithms of Needleman and Wunsch (J. Mol. Biol. 48; 443 (1970)), and "BestFit", which is based on the algorithm of Smith and Waterman (Adv. Appl. Math. 2; 482 (1981)). "Gap" and "BestFit" are part of the GCG software-package (Genetics Computer Group, 575 Science Drive, Madison, Wisconsin, USA 53711 (1991); Altschul et al., (Nucleic Acids Res. 25, 3389 (1997)), "Needle" is part of the The European Molecular Biology Open Software Suite (EMBOSS) (Trends in Genetics 16 (6), 276 (2000)). Therefore preferably the calculations to determine the percentages of sequence identity are done with the programs "Gap" or "Needle" over the whole range of the sequences. The following standard adjustments for the comparison of nucleic acid sequences were used for "Needle": matrix: EDNAFULL, Gap_penalty: 10.0, Extend_penalty: 0.5. The following standard adjustments for the comparison of nucleic acid sequences were used for "Gap": gap weight: 50, length weight: 3, average match: 10.000, average mismatch: 0.000.

For example a sequence, which is said to have 80% identity with sequence SEQ ID NO: 1 at the nucleic acid level is understood as meaning a sequence which, upon comparison with the sequence represented by SEQ ID NO: 1 by the above program "Needle" with the above parameter set, has a 80% identity. Preferably the identity is calculated on the complete length of the query sequence, for example SEQ ID NO:1.

Isogenic: organisms (e.g., plants), which are genetically identical, except that they may differ by the presence or absence of a heterologous DNA sequence.

Isolated: The term "isolated" as used herein means that a material has been removed by the hand of man and exists apart from its original, native environment and is therefore not a product of nature. An isolated material or molecule (such as a DNA molecule or enzyme) may exist in a purified form or may exist in a non-native environment such as, for example, in a transgenic host cell. For example, a naturally occurring polynucleotide or polypeptide present in a living plant is not isolated, but the same polynucleotide or polypeptide, separated from some or all of the coexisting materials in the natural system, is isolated. Such polynucleotides can be part of a vector and/or such polynucleotides or polypeptides could be part of a composition, and would be isolated in that such a vector or composition is not part of its original environment. Preferably, the term "isolated" when used in relation to a nucleic acid molecule, as in "an isolated nucleic acid sequence" refers to a nucleic acid sequence that is identified and separated from at least one contaminant nucleic acid molecule with which it is ordinarily associated in its natural source. Isolated nucleic acid molecule is nucleic acid molecule present in a form or setting that is different from that in which it is found in nature. In contrast, non-isolated nucleic acid molecules are nucleic acid molecules such as DNA and RNA, which are found in the state they exist in nature. For example, a given DNA sequence (e.g., a gene) is found on the host cell chromosome in proximity to neighboring genes; RNA sequences, such as a specific mRNA sequence encoding a specific protein, are found in the cell as a mixture with numerous other mRNAs, which encode a multitude of proteins. However, an isolated nucleic acid sequence comprising for example SEQ ID NO: 1 includes, by way of example, such nucleic acid sequences in cells which ordinarily contain SEQ ID NO:1 where the nucleic acid sequence is in a chromosomal or extrachromosomal location different from that of natural cells, or is otherwise flanked by a different nucleic acid sequence than that found in nature. The isolated nucleic acid sequence may be present in single-stranded or double-stranded form. When an isolated nucleic acid sequence is to be utilized to express a protein, the nucleic acid sequence will contain at a minimum at least a portion of the sense or coding strand (i.e., the nucleic acid sequence may be single-stranded). Alternatively, it may contain both the sense and anti-sense strands (i.e., the nucleic acid sequence may be double-stranded).

Minimal Promoter: promoter elements, particularly a TATA element, that are inactive or that have greatly reduced promoter activity in the absence of upstream activation. In the presence of a suitable transcription factor, the minimal promoter functions to permit transcription.

"Modulating the expression of a gene" in a plant as used herein means the increase, enhancement, repression or downregulation of the expression of a target gene compared to a wild-type or reference plant or part thereof to which the method of the invention has not been applied. According to the invention, the modulation of the expression of a target gene is achieved by suppression of the activity of a srRNA such as, for example, a microRNA or an enhancing RNA (RNAa). Depending on the activity of the respective srRNA, either repressing the expression of the respective target gene or activating the expression of the respective target gene, the effect of the suppression of the activity of the srRNA is either an increase or enhancement of the expression of the target gene or the repression or downregulation of the expression of the target gene. Both are covered by the term "modulating the expression of a gene".

"Naturally" when used herein in respect to a nucleic acid sequence or nucleic acid molecule or a gene means that the respective sequence or molecule is present in a wild-type plant cell, that has not been genetically modified or manipulated by man. A srRNA molecule naturally targeting a target gene means a srRNA molecule present in a wild-type plant cell, the cell has not been genetically modified or manipulated by man which is targeting a target gene naturally occurring in the respective plant cell.

Non-coding: The term "non-coding" refers to sequences of nucleic acid molecules that do not encode part or all of an expressed protein. Non-coding sequences include but are not limited to introns, enhancers, promoter regions, 3' untranslated regions, and 5' untranslated regions.

Nucleic acids and nucleotides: The terms "Nucleic Acids" and "Nucleotides" refer to naturally occurring or synthetic or artificial nucleic acid or nucleotides. The terms "nucleic acids" and "nucleotides" comprise deoxyribonucleotides or ribonucleotides or any nucleotide analogue and polymers or hybrids thereof in either single- or double-stranded, sense or antisense form. Unless otherwise indicated, a particular nucleic acid sequence also implicitly encompasses conservatively modified variants thereof (e.g., degenerate codon substitutions) and complementary sequences, as well as the sequence explicitly indicated. The term "nucleic acid" is used inter-changeably herein with "gene", "cDNA, "mRNA", "oligonucleotide," and "polynucleotide". Nucleotide analogues include nucleotides having modifications in the chemical structure of the base, sugar and/or phosphate, including, but not limited to, 5-position pyrimidine modifications, 8-position purine modifications, modifications at cytosine exocyclic amines, substitution of 5-bromo-uracil, and the like; and 2'-position sugar modifications, including but not limited to, sugar-modified ribonucleotides in which the 2'-OH is replaced by a group selected from H, OR, R, halo, SH, SR, NH2, NHR, NR2, or CN. Short hairpin RNAs (shRNAs) also can comprise non-natural elements such as non-natural bases, e.g., ionosin and xanthine, non-natural sugars, e.g., 2'-methoxy ribose, or non-natural phosphodiester linkages, e.g., methylphosphonates, phosphorothioates and peptides.

Nucleic acid sequence: The phrase "nucleic acid sequence" refers to a single or double-stranded polymer of deoxyribonucleotide or ribonucleotide bases read from the 5'- to the 3'-end. It includes chromosomal DNA, self-replicating plasmids, infectious polymers of DNA or RNA and DNA or RNA that performs a primarily structural role. "Nucleic acid sequence" also refers to a consecutive list of abbreviations, letters, characters or words, which represent nucleotides. In one embodiment, a nucleic acid can be a "probe" which is a relatively short nucleic acid, usually less than 100 nucleotides in length. Often a nucleic acid probe is from about 50 nucleotides in length to about 10 nucleotides in length. A "target region" of a nucleic acid is a portion of a nucleic acid that is identified to be of interest. A "coding region" of a nucleic acid is the portion of the nucleic acid, which is transcribed and translated in a sequence-specific manner to produce into a particular polypeptide or protein when placed under the control of appropriate regulatory sequences. The coding region is said to encode such a polypeptide or protein.

Oligonucleotide: The term "oligonucleotide" refers to an oligomer or polymer of ribonucleic acid (RNA) or deoxyribonucleic acid (DNA) or mimetics thereof, as well as oligonucleotides having non-naturally-occurring portions which function similarly. Such modified or substituted oligonucleotides are often preferred over native forms because of desirable properties such as, for example, enhanced cellular uptake, enhanced affinity for nucleic acid target and increased stability in the presence of nucleases. An oligonucleotide preferably includes two or more nucleomonomers covalently coupled to each other by linkages (e.g., phosphodiesters) or substitute linkages.

Overhang: An "overhang" is a relatively short single-stranded nucleotide sequence on the 5'- or 3'-hydroxyl end of a double-stranded oligonucleotide molecule (also referred to as an "extension," "protruding end," or "sticky end"). Plant: is generally understood as meaning any eukaryotic single-or multi-celled organism or a cell, tissue, organ, part or propagation material (such as seeds or fruit) of same which is capable of photosynthesis. Included for the purpose of the invention are all genera and species of higher and lower plants of the Plant Kingdom. Annual, perennial, monocotyledonous and dicotyledonous plants are preferred. The term includes the mature plants, seed, shoots and seedlings and their derived parts, propagation material (such as seeds or microspores), plant organs, tissue, protoplasts, callus and other cultures, for example cell cultures, and any other type of plant cell grouping to give functional or structural units. Mature plants refer to plants at any desired developmental stage beyond that of the seedling. Seedling refers to a young immature plant at an early developmental stage. Annual, biennial, monocotyledonous and dicotyledonous plants are preferred host organisms for the generation of transgenic plants. The expression of genes is furthermore advantageous in all ornamental plants, useful or ornamental trees, flowers, cut flowers, shrubs or lawns. Plants which may be mentioned by way of example but not by limitation are angiosperms, bryophytes such as, for example, Hepaticae (liverworts) and Musci (mosses); Pteridophytes such as ferns, horsetail and club mosses; gymnosperms such as conifers, cycads, ginkgo and Gnetatae; algae such as Chlorophyceae, Phaeophpyceae, Rhodophyceae, Myxophyceae, Xanthophyceae, Bacillariophyceae (diatoms), and Euglenophyceae. Preferred are plants which are used for food or feed purpose such as the families of the Leguminosae such as pea, alfalfa and soya; Gramineae such as rice, maize, wheat, barley, sorghum, millet, rye, triticale, or oats; the family of the Umbelliferae, especially the genus Daucus, very especially the species carota (carrot) and Apium, very especially the species Graveolens dulce (celery) and many others; the family of the Solanaceae, especially the genus Lycopersicon, very especially the species esculentum (tomato) and the genus Solanum, very especially the species tuberosum (potato) and melongena (egg plant), and many others (such as tobacco); and the genus Capsicum, very especially the species annuum (peppers) and many others; the family of the Leguminosae, especially the genus Glycine, very especially the species max (soybean), alfalfa, pea, lucerne, beans or peanut and many others; and the family of the Cruciferae (Brassicacae), especially the genus Brassica, very especially the species napus (oil seed rape), campestris (beet), oleracea cv Tastie (cabbage), oleracea cv Snowball Y (cauliflower) and oleracea cv Emperor (broccoli); and of the genus Arabidopsis, very especially the species thaliana and many others; the family of the Compositae, especially the genus Lactuca, very especially the species sativa (lettuce) and many others; the family of the Asteraceae such as sunflower, Tagetes, lettuce or Calendula and many other; the family of the Cucurbitaceae such as melon, pumpkin/squash or zucchini, and linseed. Further preferred are cotton, sugar cane, hemp, flax, chillies, and the various tree, nut and wine species.

Polypeptide: The terms "polypeptide", "peptide", "oligopeptide", "polypeptide", "gene product", "expression product" and "protein" are used interchangeably herein to refer to a polymer or oligomer of consecutive amino acid residues.

Pre-protein: Protein, which is normally targeted to a cellular organelle, such as a chloroplast, and still comprising its transit peptide.

Primary transcript: The term "primary transcript" as used herein refers to a premature RNA transcript of a gene. A "primary transcript" for example still comprises introns and/or is not yet comprising a polyA tail or a cap structure and/or is missing other modifications necessary for its correct function as transcript such as for example trimming or editing.

Promoter: The terms "promoter", or "promoter sequence" are equivalents and as used herein, refer to a DNA sequence which when ligated to a nucleotide sequence of interest is capable of controlling the transcription of the nucleotide sequence of interest into RNA. Such promoters can for example be found in the following public databases http://www.grassius.org/grasspromdb.html, http://mendel.cs.rhul.ac.uk/mendel.php?topic=plantprom, http:l/ppdb.gene.nagoya-u.ac.jp/cgi-binlindex.cgi. Promoters listed there may be addressed with the methods of the invention and are herewith included by reference. A promoter is located 5' (i.e., upstream), proximal to the transcriptional start site of a nucleotide sequence of interest whose transcription into mRNA it controls, and provides a site for specific binding by RNA polymerase and other transcription factors for initiation of transcription. Said promoter comprises for example the at least 10 kb, for example 5 kb or 2 kb proximal to the transcription start site. It may also comprise the at least 1500 bp proximal to the transcriptional start site, preferably the at least 1000 bp, more preferably the at least 500 bp, even more preferably the at least 400 bp, the at least 300 bp, the at least 200 bp or the at least 100 bp. In a further preferred embodiment, the promoter comprises the at least 50 bp proximal to the transcription start site, for example, at least 25 bp. The promoter does not comprise exon and/or intron regions or 5' untranslated regions. The promoter may for example be heterologous or homologous to the respective plant. A polynucleotide sequence is "heterologous to" an organism or a second polynucleotide sequence if it originates from a foreign species, or, if from the same species, is modified from its original form. For example, a promoter operably linked to a heterologous coding sequence refers to a coding sequence from a species different from that from which the promoter was derived, or, if from the same species, a coding sequence which is not naturally associated with the promoter (e.g. a genetically engineered coding sequence or an allele from a different ecotype or variety). Suitable promoters can be derived from genes of the host cells where expression should occur or from pathogens for this host cells (e.g., plants or plant pathogens like plant viruses). A plant specific promoter is a promoter suitable for regulating expression in a plant. It may be derived from a plant but also from plant pathogens or it might be a synthetic promoter designed by man. If a promoter is an inducible promoter, then the rate of transcription increases in response to an inducing agent. Also, the promoter may be regulated in a tissue-specific or tissue preferred manner such that it is only or predominantly active in transcribing the associated coding region in a specific tissue type(s) such as leaves, roots or meristem. The term "tissue specific" as it applies to a promoter refers to a promoter that is capable of directing selective expression of a nucleotide sequence of interest to a specific type of tissue (e.g., petals) in the relative absence of expression of the same nucleotide sequence of interest in a different type of tissue (e.g., roots). Tissue specificity of a promoter may be evaluated by, for example, operably linking a reporter gene to the promoter sequence to generate a reporter construct, introducing the reporter construct into the genome of a plant such that the reporter construct is integrated into every tissue of the resulting transgenic plant, and detecting the expression of the reporter gene (e.g., detecting mRNA, protein, or the activity of a protein encoded by the reporter gene) in different tissues of the transgenic plant. The detection of a greater level of expression of the reporter gene in one or more tissues relative to the level of expression of the reporter gene in other tissues shows that the promoter is specific for the tissues in which greater levels of expression are detected. The term "cell type specific" as applied to a promoter refers to a promoter, which is capable of directing selective expression of a nucleotide sequence of interest in a specific type of cell in the relative absence of expression of the same nucleotide sequence of interest in a different type of cell within the same tissue. The term "cell type specific" when applied to a promoter also means a promoter capable of promoting selective expression of a nucleotide sequence of interest in a region within a single tissue. Cell type specificity of a promoter may be assessed using methods well known in the art, e.g., GUS activity staining, GFP protein or immunohistochemical staining. The term "constitutive" when made in reference to a promoter or the expression derived from a promoter means that the promoter is capable of directing transcription of an operably linked nucleic acid molecule in the absence of a stimulus (e.g., heat shock, chemicals, light, etc.) in the majority of plant tissues and cells throughout substantially the entire lifespan of a plant or part of a plant. Typically, constitutive promoters are capable of directing expression of a transgene in substantially any cell and any tissue.

Promoter specificity: The term "specificity" when referring to a promoter means the pattern of expression conferred by the respective promoter. The specificity describes the tissues and/or developmental status of a plant or part thereof, in which the promoter is conferring expression of the nucleic acid molecule under the control of the respective promoter. Specificity of a promoter may also comprise the environmental conditions, under which the promoter may be activated or down-regulated such as induction or repression by biological or environmental stresses such as cold, drought, wounding or infection.

Purified: As used herein, the term "purified" refers to molecules, either nucleic or amino acid sequences that are removed from their natural environment, isolated or separated. "Substantially purified" molecules are at least 60% free, preferably at least 75% free, and more preferably at least 90% free from other components with which they are naturally associated. A purified nucleic acid sequence may be an isolated nucleic acid sequence.

Recombinant: The term "recombinant" with respect to nucleic acid molecules refers to nucleic acid molecules produced by recombinant DNA techniques. Recombinant nucleic acid molecules may also comprise molecules, which as such does not exist in nature but are modified, changed, mutated or otherwise manipulated by man. Preferably, a "recombinant nucleic acid molecule" is a non-naturally occurring nucleic acid molecule that differs in sequence from a naturally occurring nucleic acid molecule by at least one nucleic acid. A "recombinant nucleic acid molecule" may also comprise a "recombinant construct" which comprises, preferably operably linked, a sequence of nucleic acid molecules not naturally occurring in that order. Preferred methods for producing said recombinant nucleic acid molecule may comprise cloning techniques, directed or non-directed mutagenesis, synthesis or recombination techniques.

"Repress" or "downregulate" the expression of a nucleic acid molecule in a plant cell are used equivalently herein and mean that the level of expression of the nucleic acid molecule in a plant, part of a plant or plant cell after applying a method of the present invention is lower than its expression in the plant, part of the plant or plant cell before applying the method, or compared to a reference plant lacking a recombinant nucleic acid molecule of the invention. For example, the reference plant is comprising the same construct which is only lacking the respective precursor molecule. The term "repressed" or "downregulated" as used herein are synonymous and means herein lower, preferably significantly lower expression of the nucleic acid molecule to be expressed. As used herein, a "repression" or "downregulation" of the level of an agent such as a protein, mRNA or RNA means that the level is reduced relative to a substantially identical plant, part of a plant or plant cell grown under substantially identical conditions, lacking a recombinant nucleic acid molecule of the invention, for example lacking the region complementary to at least a part of the precursor molecule of the srRNA, the recombinant construct or recombinant vector of the invention. As used herein, "repression" or "downregulation" of the level of an agent, such as for example a preRNA, mRNA, rRNA, tRNA, snoRNA, snRNA expressed by the target gene and/or of the protein product encoded by it, means that the amount is reduced 10% or more, for example 20% or more, preferably 30% or more, more preferably 50% or more, even more preferably 70% or more, most preferably 80% or more for example 90% relative to a cell or organism lacking a recombinant nucleic acid molecule of the invention. The repression or downregulation can be determined by methods with which the skilled worker is familiar. Thus, the enhancement or increase of the nucleic acid or protein quantity can be determined for example by an immunological detection of the protein. Moreover, techniques such as protein assay, fluorescence, Northern hybridization, nuclease protection assay, reverse transcription (quantitative RT-PCR), ELISA (enzyme-linked immunosorbent assay), Western blotting, radioimmunoassay (RIA) or other immunoassays and fluorescence-activated cell analysis (FACS) can be employed to measure a specific protein or RNA in a plant or plant cell. Depending on the type of the induced protein product, its activity or the effect on the phenotype of the organism or the cell may also be determined. Methods for determining the protein quantity are known to the skilled worker. Examples, which may be mentioned, are: the micro-Biuret method (Goa J (1953) Scand J Clin Lab Invest 5:218-222), the Folin-Ciocalteau method (Lowry OH et al. (1951) J Biol Chem 193:265-275) or measuring the absorption of CBB G-250 (Bradford MM (1976) Analyt Biochem 72:248-254). As one example for quantifying the activity of a protein, the detection of luciferase activity is described in the Examples below.

Sense: The term "sense" is understood to mean a nucleic acid molecule having a sequence which is complementary or identical to a target sequence, for example a sequence which binds to a protein transcription factor and which is involved in the expression of a given gene. According to a preferred embodiment, the nucleic acid molecule comprises a gene of interest and elements allowing the expression of the said gene of interest.

Significant increase or decrease: An increase or decrease, for example in enzymatic activity or in gene expression, that is larger than the margin of error inherent in the measurement technique, preferably an increase or decrease by about 2-fold or greater of the activity of the control enzyme or expression in the control cell, more preferably an increase or decrease by about 5-fold or greater, and most preferably an increase or decrease by about 10-fold or greater.

Small regulating RNA molecules: "small regulating RNA molecules" or srRNA molecules are understood as molecules consisting of nucleic acids or derivatives thereof. They may be double-stranded or single-stranded and are between about 15 and about 30 bp, for example between 15 and 30 bp, more preferred between about 19 and about 26 bp, for example between 19 and 26 bp, even more preferred between about 20 and about 25 bp for example between 20 and 25 bp. In an especially preferred embodiment the oligonucleotides are between about 21 and about 24 bp, for example between 21 and 24 bp. In a most preferred embodiment, the small nucleic acid molecules are about 21 bp and about 24 bp, for example 21 bp and 24 bp. SrRNA molecules may be derived from larger precursor RNA molecules that comprise such srRNA molecules and that are processed in vitro or in vivo and release upon processing such srRNA molecules. SrRNA molecules may also be synthesized by man.

Substantially complementary: In its broadest sense, the term "substantially complementary", when used herein with respect to a nucleotide sequence in relation to a reference or target nucleotide sequence, means a nucleotide sequence having a percentage of identity between the substantially complementary nucleotide sequence and the exact complementary sequence of said reference or target nucleotide sequence of at least 60%, more desirably at least 70%, more desirably at least 80% or 85%, preferably at least 90%, more preferably at least 93%, still more preferably at least 95% or 96%, yet still more preferably at least 97% or 98%, yet still more preferably at least 99% or most preferably 100% (the later being equivalent to the term "identical" in this context). Preferably identity is assessed over a length of at least 19 nucleotides, preferably at least 50 nucleotides, more preferably the entire length of the nucleic acid sequence to said reference sequence (if not specified otherwise below). Sequence comparisons are carried out using default GAP analysis with the University of Wisconsin GCG, SEQWEB application of GAP, based on the algorithm of Needleman and Wunsch (Needleman and Wunsch (1970) J Mol. Biol. 48: 443-453; as defined above). A nucleotide sequence "substantially complementary " to a reference nucleotide sequence hybridizes to the reference nucleotide sequence under low stringency conditions, preferably medium stringency conditions, most preferably high stringency conditions (as defined above).

Suppressing the activity of a srRNA molecule such as microRNA molecule, ta-siRNA or RNAa molecule, means that the regulatory function of said srRNA is reduced or lost and hence the amount of transcript of a target gene targeted by the respective srRNA in a plant, part of a plant or plant cell after applying a method of the present invention is changed compared to a wild-type or reference plant. Depending on whether the activity of the respective srRNA is the downregulation or activation of its target gene, the plant or part thereof comprises a higher or lower amount of transcript than the amount of transcript of said target gene in the plant, part of the plant or plant cell before applying the method of the invention, or compared to a reference plant, part of the plant or plant cell lacking a recombinant nucleic acid molecule of the invention.

In case, the activity of the respective srRNA is the downregulation or repression of the respective target gene, the amount of transcript is higher, preferably significantly higher in the plant comprising a recombinant nucleic acid of the invention. As used herein, an higher amount of a transcript such as an mRNA or RNA means that the level is higher relative to a substantially identical plant, part of a plant or plant cell grown under substantially identical conditions, lacking a recombinant nucleic acid molecule of the invention, for example lacking, the recombinant construct or recombinant vector of the invention. As used herein, a higher amount of a transcript, such as for example a preRNA, mRNA, rRNA, tRNA, snoRNA, snRNA and the like expressed by the target gene, means that the amount is increased 50% or more, for example 100% or more, preferably 200% or more, more preferably 5 fold or more, even more preferably 10 fold or more, most preferably 20 fold or more for example 50 fold relative to a cell or organism lacking a recombinant nucleic acid molecule of the invention.

In case the activity of the respective srRNA is the activation of the respective target gene, the amount of transcript is lower, preferably significantly lower in the plant comprising a recombinant nucleic acid of the invention. As used herein, a lower amount of a transcript such as an mRNA or RNA means that the level is lower relative to a substantially identical plant, part of a plant or plant cell grown under substantially identical conditions, lacking a recombinant nucleic acid molecule of the invention, for example lacking, the recombinant construct or recombinant vector of the invention. As used herein, a lower amount of a transcript, such as for example a preRNA, mRNA, rRNA, tRNA, snoRNA, snRNA and the like expressed by the target gene, means that the amount is reduced 10% or more, for example 20% or more, preferably 30% or more, more preferably 50% or more, even more preferably 70% or more, most preferably 80% or more for example 90% relative to a cell or organism lacking a recombinant nucleic acid molecule of the invention.

The enhancement or increase or decrease or suppression of the transcript can be determined by methods with which the skilled worker is familiar. Thus, the enhancement or increase or decrease or suppression of the nucleic acid quantity can be determined for example by techniques such as Northern hybridization, nuclease protection assay, reverse transcription (quantitative RT-PCR).

Transgene: The term "transgene" as used herein refers to any nucleic acid sequence, which is introduced into the genome of a cell by experimental manipulations. A transgene may be an "endogenous DNA sequence," or a "heterologous DNA sequence" (i.e., "foreign DNA"). The term "endogenous DNA sequence" refers to a nucleotide sequence, which is naturally found in the cell into which it is introduced so long as it does not contain some modification (e.g., a point mutation, the presence of a selectable marker gene, etc.) relative to the naturally-occurring sequence.

Transgenic: The term transgenic when referring to an organism means transformed, preferably stably transformed, with a recombinant DNA molecule that preferably comprises a suitable promoter operatively linked to a DNA sequence of interest.

Vector: As used herein, the term "vector" refers to a nucleic acid molecule capable of transporting another nucleic acid molecule to which it has been linked. One type of vector is a genomic integrated vector, or "integrated vector", which can become integrated into the chromosomal DNA of the host cell. Another type of vector is an episomal vector, i.e., a nucleic acid molecule capable of extrachromosomal replication. Vectors capable of directing the expression of genes to which they are operatively linked are referred to herein as "expression vectors".

In the present specification, "plasmid" and "vector" are used interchangeably unless otherwise clear from the context. Expression vectors designed to produce RNAs as described herein in vitro or in vivo may contain sequences recognized by any RNA polymerase, including mitochondrial RNA polymerase, RNA pol 1, RNA pol II, and RNA pol III. These vectors can be used to transcribe the desired RNA molecule in the cell according to this invention. A plant transformation vector is to be understood as a vector suitable in the process of plant transformation.

Wild-type: The term "wild-type", "natural" or "natural origin" means with respect to an organism, polypeptide, or nucleic acid sequence, that said organism is naturally occurring or available in at least one naturally occurring organism which is not changed, mutated, or otherwise manipulated by man.

### EXAMPLES

### Chemicals and common methods

Unless indicated otherwise, cloning procedures carried out for the purposes of the present invention including restriction digest, agarose gel electrophoresis, purification of nucleic acids, Ligation of nucleic acids, transformation, selection and cultivation of bacterial cells were performed as described (Sambrook et al., 1989). Sequence analyses of recombinant DNA were performed with a laser fluorescence DNA sequencer (Applied Biosystems, Foster City, CA, USA) using the Sanger technology (Sanger et al., 1977). Unless described otherwise, chemicals and reagents were obtained from Sigma Aldrich (Sigma Aldrich, St. Louis, USA), from Promega (Madison, WI, USA), Duchefa (Haarlem, The Netherlands) or Invitrogen (Carlsbad, CA, USA). Restriction endonucleases were from New England Biolabs (Ipswich, MA, USA) or Roche Diagnostics GmbH (Penzberg, Germany). Oligonucleotides were synthesized by Eurofins MWG Operon (Ebersberg, Germany).

### Example 1: Suppressing endogenous miRNA activity in monocots via target mimics

Maize and rice databases were Blastn searched for sequences containing a sequence similar to the motif TAGGGCAACTTNNNTCCTTTGGCA (SEQ ID NO. 30), where N is any nucleotide base. Maize and rice sequences were identified from the database containing sequences similar to this motif.

**Table 1. Endogenous target mimicry sequences from maize and rice**

| Sequence name | source | sequence |
|---|---|---|
| ZM06MC40391_TD14607 6974 rc | Maize | AGGCTTGCTATAGCTGGTCCATGGC ACCATACATGTAAGCACGCACACAG GCACACACACACACGCACGCAATGA TCTACGTATCTAGCAGCAGCTTATCA TGTCGTCATCATGCATGCATGGCCG ACGGAGGTCGTCATCTTATCTGGGA GCGTGTGTGTCTTGGCAATGGGAAG CTGCATGCGCCTCTCGGGCGTCGG CGCGTCGGCGCCTAGCTGTAGGGC GGCGTGCCATAGAGCTGCCTCCTG CCGCTCACACCATGCTGTTGACGAG GACTGATGGTGGTCATGGCCTCTCG GCGTCGGTGGCGGCGGCGCCGGC GCCGAGTTTTACCTCTCTACTAAGG TAGGGCAACTTGTATCCTTTGGCAA TTGTTCTCATCTATCTGGGTCTGTCT GTTGGCTGCCCGGTGACGGTATAC GGTGATGTTCTAATAGTACTCAATTG GTCTTGGATCGGAGTTCATGCTACG GCTCCTCTGTTATATATTACACGGCT GACGGCTCCTTATTAATGTGTCCGT TGATGATGTCATTAATAATATGAATC TGATATTGTATAAAAAAAAAAAAAA |
| | Maize | |
| sequence shown is the reverse complement of ZM06MC40391_TD14607 6974 | Mefi Version 06 (SEQ ID NO.1) | |
| OS07MC07755_33971786 | Rice | AGGGGGTGTGTACTGCTGATTCCTC ATGCGTCGTCGGAGATCGAGAGGT |
| | Hyseq_ri ce_mefi_ v07.nt (SEQ ID NO.2) | GATGCATGCTGCGAACGGCGAGCG GCGGCGGCGGCGACAGGAGGTCG GCGGCGAGCTCCGGCGTGCTGGCT GGCATCCGGCGATCGACGACGGCG ACCGAGGGAGGGAGGAAGCTATAG CTAGGATGGTCATCATGTGATTAATT CAGTGATGTGTCCTAATTAAGTAGCT TCTTGCTTGCTAGCAGGGCAATTTC AATCCTTTGGCATCTGTAACCTTTTT CTTTCTTTCTGAAGTAAACCTTAATA ACCCAGCTATGTTCTCTTATGATGTT CTGAACAAATACAACAAATTAGGCAT ACAGATTCAGATACAATAGCACTTGA GTTTGTATCTAGTCGTTTGTCAAGAA CCAAGACACATTTCTTG |

The 24 nt sequence having a motif similar to TAGGGCAACTTNNNTCCTTTGGCA (SEQ ID NO. 30) is underlined in each sequence.

### Artificial miR166 target mimic.

To create a sequence that when expressed in maize will act as a target for endogenous miR166 miRNAs, sequence ZM06MC40391_TD146076974 rc (Table 1) was modified as follows:
1. The sequence
   TACTAAGGTAGGGCAACTTGTATCCTTTGGCAATTGTTCTCATC (SEQ ID NO. 31) spanning nucleotides 339-382 of ZM06MC40391_TD146076974 rc and including the motif similar to SEQ ID NO. 30 (underlined) was replaced with sequence
   TCCGGAGGGGGGAATGAAGAAACCTGGTCCGAATTGCGGCCGC (SEQ ID NO. 32) containing a miR166 target site with a BspEl site (TCCGGA) 5' of the miR166 targets site and a Notl site (GCGGCCGC) 3' of the miR166 target site.
2. The 14 nt poly(A) tail spanning nucleotides 561-574 in
   ZM06MC40391_TD146076974 rc was deleted.

**Table 2. The final miR166 target sequence (Zm miR166-trap) with the miR166 target site underlined.**

| Sequence name | sequence |
|---|---|
| Zm miR166-trap(SEQ ID NO. 33) | AGGCTTGCTATAGCTGGTCCATGGCACCATACATGTAAGCA CGCACACAGGCACACACACACACGCACGCAATGATCTACGT ATCTAGCAGCAGCTTATCATGTCGTCATCATGCATGCATGGC CGACGGAGGTCGTCATCTTATCTGGGAGCGTGTGTGTCTTG GCAATGGGAAGCTGCATGCGCCTCTCGGGCGTCGGCGCGT CGGCGCCTAGCTGTAGGGCGGCGTGCCATAGAGCTGCCTC CTGCCGCTCACACCATGCTGTTGACGAGGACTGATGGTGGT CATGGCCTCTCGGCGTCGGTGGCGGCGCCGGCGCCG AGTTTTACCTCTCTCCGGAGGGGGGAATGAAGAAACCTGGT CCGAATTGCGGCCGCTATCTGGGTCTGTCTGTTGGCTGCCC GGTGACGGTATACGGTGATGTTCTAATAGTACTCAATTGGTC TTGGATCGGAGTTCATGCTACGGCTCCTCTGTTATATATTAC ACGGCTGACGGCTCCTTATTAATGTGTCCGTTGATGATGTCA TTAATAATATGAATCTGATATTGTAT |

The miR166 target site is complementary to known maize miR166 family members in maize with a 3 nt bulge in the target sequence between bases 10 and 11 from the 5' end of the miRNA. miRNA sequences were obtained from miRbase (www.mirbase.org). RNA sequences are shown.

| | |
|---|---|
| Target site in Zm miR166-trap | 5'GGGGAAUGAAGAAACCUGGUCCGA 3' |
| (SEQ ID NO. 34) | |
| zma-MIR166k and j | 3'UCCCUAACUUC GGACCAGGCU 5' |
| (SEQ ID NO. 35) | |
| zma-MIR1661 and m | 3'CUCCUUACUUC GGACCAGGCU 5' |
| (SEQ ID NO. 36) | |
| zma-MIR166h, e, I, f, g, b, c, d | 3' CCCUUACUUC GGACCAGGCU 5' |
| (SEQ ID NO. 37) | |
| zma-MIR166a | 3'CCCCUUACUUC GGACCAGGCU 5' |
| (SEQ ID NO. 38) | |

Zm miR166-trap was synthesized and cloned into a binary vector that contained the maize transformation selectable marker ZmAHASL2_A122(At)T/S653(At)N to create plasmid RTP2149-1qcz (SEQ ID NO.3). In RTP2149-1qcz, Zm miR166-trap was expressed under control of the ScBV[mm247] promoter plus i-Met1-1 [mm369] intron and NOS terminator and ZmAHASL2_A122(At)T/S653(At)N was expressed under control of the p-Ubi promoter and t-X112-3'/UTR terminator.

Partial sequence of RTP2149-1 qcz from bases 5386 to 8250, including the elements p-SCBV(mm247) (bases 5386-6806), i-met1-1(mm369) (bases 6835-7417), c-Zm miR166-TRAP (bases 7426-7984), and t-NOS (bases 7998-8250) is presented as SEQ ID NO. 39.

Generation of To RTP2149-1 qcz plants:
RTP2149-1 qcz and the control construct RCB958-1 qcz (SEQ ID NO. 4) were transformed into maize J553 x (Hill x Ai88) embryos. RCB958-1qcz is a maize binary vector similar to RTP2149-1 qcz but instead expresses GUS. In RCB958-1 qcz, GUS was expressed under control of the ScBV[mm247] promoter plus i-Met1-1[mm369] intron and NOS terminator and
ZmAHASL2_A122(At)T/S653(At)N was expressed under control of the p-Ubi promoter and t-XI12-3'/UTR terminator.

RTP2149-1 qcz and RCB958-1 qcz transformed maize were grown in the greenhouse, leaf harvested 11 or 17 days after transfer to big pots, and RNA extracted. Rld1 (rolled leaf 1) RNA has been demonstrated to be a target of miR166 in maize (Nature. 2004. 428: 84-88). Taqman probes were designed for rld1 and the expression level of Rld was determined by quantitative RT-PCR in the RNA isolated from each leaf sample (tables 3-6). Three quantitative RT-PCR technical reps were performed for each RNA sample and the average of the 3 technical reps for each plant and the overall group average of relative rld1 expression are reported in tables 3-6.

**Table 3. Relative expression of rld1 in transgenic events carrying RTP2149-1 qcz**

| | | | relative rld1 expression |
|---|---|---|---|
| Construct Name | Plant ID | days from big pots to harvest | average of 3 technical reps |
| RTP2149-1qcz | 106386201 | 17 | 3.9 |
| RTP2149-1qcz | 106386231 | 17 | 5.4 |
| RTP2149-1qcz | 106386261 | 17 | 2.1 |
| RTP2149-1qcz | 106386271 | 17 | 1.4 |
| RTP2149-1qcz | 106386291 | 17 | 3.5 |
| RTP2149-1qcz | 106386301 | 17 | 6.6 |
| RTP2149-1qcz | 106424471 | 17 | 3.7 |
| | | avg | 3.8 |

**Table 4. Relative expression of rld1 in transgenic events carrying RCB958-1 qcz**

| | | | relative rld1 expression |
|---|---|---|---|
| Construct Name | Plant ID | days from big pots to harvest | average of 3 technical reps |
| RCB958-1qcz | 106386041 | 17 | 1.0 |
| RCB958-1qcz | 106386061 | 17 | 3.4 |
| RCB958-1qcz | 106386091 | 17 | 2.5 |
| RCB958-1qcz | 106386101 | 17 | 1.8 |
| RCB958-1qcz | 106386141 | 17 | 1.1 |
| RCB958-1qcz | 106386161 | 17 | 1.3 |
| | | avg | 1.8 |

**Table 5. Relative expression of rld1 in transgenic events carrying RTP2149-1 qcz**

| | | | relative rld1 expression |
|---|---|---|---|
| Construct Name | Plant ID | days from big pots to harvest | average of 3 technical reps |
| RTP2149-1qcz | 106386241 | 10 | 3.5 |
| RTP2149-1qcz | 106386251 | 10 | 3.3 |
| RTP2149-1qcz | 106386331 | 10 | 0.7 |
| RTP2149-1qcz | 106386351 | 10 | 5.9 |
| RTP2149-1qcz | 106395911 | 10 | 3.3 |
| RTP2149-1qcz | 106424461 | 10 | 2.9 |
| | | avg | 3.3 |

**Table 6. Relative expression of rld1 in transgenic events carrying RTP958-1 qcz**

| | | | relative rld1 expression |
|---|---|---|---|
| Construct Name | Plant ID | days from big pots to harvest | average of 3 technical reps |
| RCB958-1qcz | 106386081 | 10 | 1.3 |
| RCB958-1qcz | 106386151 | 10 | 0.6 |
| | | avg | 0.9 |

The data from tables 3-6 demonstrate a increase in expression of rld1 in transgenic plants expressing Zm miR166-TRAP (RTP2149-1qcz) as expected if the activity of miR166 is suppressed with respect to rld1.

Rld expression in segregating T1 seedlings.

The RTP2149-1 qcz To transgenic plant 106386231 (table 3) was shown to have a single copy insertion of Zm miR166-TRAP by Taqman copy number analysis. This plant was self-pollinated and the resulting T₁ seeds planted. Zygosity of each seedling was determined by Taqman copy number analysis as homozygous for Zm miR166-TRAP, hemizygous for Zm miR166-TRAP, or null.

The entire 3^{rd} leaf, counting from bottom, of 15 homozygous, 23 hemizygous, and 26 null 13 day old seedlings was harvested, RNA extracted, and Rld1 expression level determined by Taqman quantitative RT-PCR. The least squares mean for each population is reported in table 7. The p-values between the null populations and hemizygous and homozygous populations are reported in table 8 demonstrating the higher expression levels of Rld1 in plants expressing Zm miR166-trap vs. nulls is significant.

**Table 7. Relative expression of rld1 in transgenic events**

| | Relative Rld1 expression | |
|---|---|---|
| group | Least squares mean | Standard Error |
| null | 1.00 | 0.14 |
| hemizygous | 2.21 | 0.14 |
| homozygous | 2.54 | 0.18 |

**Table 8. Relative expression of rld1 in transgenic events**

| | pvalue |
|---|---|
| null vs hemizygous | 7.22E-08 |
| null vs homozygous | 4.05E-09 |

Target mimic for suppression of multiple miRNAs:

To create a sequence that when expressed in a monocot will act as a target for mutliple endogenous small regulating RNAs, two or more Zm miR166-trap sequences (Table 2) can be expressed in tandem as one transcript with each sequence containing a target site for a different small regulating RNA. In table 9, is a tandem construct with target sites for miR166 (first 24nt sequence underlined) and miR159 (second 24nt sequence underlined).

**Table 9. Zm miR166-trap/Zm miR-159 trap sequence.**

| Sequence name | sequence |
|---|---|
| Zm miR166-trap/Zm miR159-trap (SEQ ID NO 5) | AGGCTTGCTATAGCTGGTCCATGGCACCATACATG TAAGCACGCACACAGGCACACACACACACGCACGC AATGATCTACGTATCTAGCAGCAGCTTATCATGTCG TCATCATGCATGCATGGCCGACGGAGGTCGTCATC TTATCTGGGAGCGTGTGTGTCTTGGCAATGGGAAG CTGCATGCGCCTCTCGGGCGTCGGCGCGTCGGCG CCTAGCTGTAGGGCGGCGTGCCATAGAGCTGCCT CCTGCCGCTCACACCATGCTGTTGACGAGGACTGA TGGTGGTCATGGCCTCTCGGCGTCGGTGGCGGCG GCGCCGGCGCCGAGTTTTACCTCTCTCCGGAGGG GGGAATGAAGAAACCTGGTCCGAATTGCGGCCGCT ATCTGGGTCTGTCTGTTGGCTGCCCGGTGACGGTA TACGGTGATGTTCTAATAGTACTCAATTGGTCTTGG ATCGGAGTTCATGCTACGGCTCCTCTGTTATATATT ACACGGCTGACGGCTCCTTATTAATGTGTCCGTTG ATGATGTCATTAATAATATGAATCTGATATTGTATAG GCTTGCTATAGCTGGTCCATGGCACCATACATGTAA GCACGCACACAGGCACACACACACACGCACGCAAT GATCTACGTATCTAGCAGCAGCTTATCATGTCGTCA |
| | TCATGCATGCATGGCCGACGGAGGTCGTCATCTTA TCTGGGAGCGTGTGTGTCTTGGCAATGGGAAGCTG CATGCGCCTCTCGGGCGTCGGCGTCGGCGCCT AGCTGTAGGGCGGCGTGCCATAGAGCTGCCTCCT GCCGCTCACACCATGCTGTTGACGAGGACTGATGG TGGTCATGGCCTCTCGGCGTCGGTGGCGGCGGCG CCGGCGCCGAGTTTTACCTCTCTCCGGAGGCGGA GCTCCCTCACTCAATCCAAAATTGCGGCCGCTATCT GGGTCTGTCTGTTGGCTGCCCGGTGACGGTATACG GTGATGTTCTAATAGTACTCAATTGGTCTTGGATCG GAGTTCATGCTACGGCTCCTCTGTTATATATTACAC GGCTGACGGCTCCTTATTAATGTGTCCGTTGATGAT GTCATTAATAATATGAATCTGATATTGTAT |

The miR159 target site in Zm miR159-trap is complementary to known maize miR159 family members with a 3 nt bulge in the target sequence between bases 10 and 11 from the 5' end of the miRNA. miRNA sequences were obtained from miRbase (www.mirbase.org). RNA sequences are shown.

| | |
|---|---|
| Target site in Zm miR166-trap | 5'CGGAGCUCCCUCACUCAAUCCAAA 3' |
| (SEQ ID NO. 40) | |
| zma-miR159a | 3'GUCUCGAGGGA AGUUAGGUUU 5' |
| (SEQ ID NO. 41) | |
| zma-miR159b | 3'GUCUCGAGGGA AGUUAGGUUU 5' |
| (SEQ ID NO. 42) | |
| zma-miR159c | 3'UCCUCGAGGGA AGUUAGGUUC 5' |
| (SEQ ID NO. 43) | |
| zma-miR159d | 3'UCCUCGAGGGA AGUUAGGUUC 5' |
| (SEQ ID NO. 44) | |

A second example to suppress two or more small regulating RNAs in a monocot is to transcribe two or more non-identical sequences in tandem, each containing a target sequence for a different small regulating RNA. An example of this type of tandem construct, Zm miR166-trap / Os miR159-trap Z, is in table 10. Zm miR166-trap / Os miR159-trap, combines the sequence Zm miR166-trap (sequence in table 2) fused with sequence OS07MC07755_33971786 (table 1) modified to contain a miR159 target site. The OS07MC07755_33971786 sequence is modified to contain a miR159 target site by deleting the 24nt miR399 target site underlined in table1 and replacing it with a 24nt miR159 target site identical to the miR159 target site in the Zm miR166-trap / Zm miR159-trap sequence in table 9. The miR159 target site is the second underlined sequence in table 10.

**Table 10. Zm miR166-trap / Os miR159-trap**

| Sequence name | sequence |
|---|---|
| Zm miR166-trap / Os miR159-trap (SEQ ID NO.6) | AGGCTTGCTATAGCTGGTCCATGGCACCATAC ATGTAAGCACGCACACAGGCACACACACACAC GCACGCAATGATCTACGTATCTAGCAGCAGCT TATCATGTCGTCATCATGCATGCATGGCCGAC GGAGGTCGTCATCTTATCTGGGAGCGTGTGT GTCTTGGCAATGGGAAGCTGCATGCGCCTCT CGGGCGTCGGCGCGTCGGCGCCTAGCTGTA GGGCGGCGTGCCATAGAGCTGCCTCCTGCCG CTCACACCATGCTGTTGACGAGGACTGATGGT GGTCATGGCCTCTCGGCGTCGGTGGCGGCG GCGCCGGCGCCGAGTTTTACCTCTCTCCGGA GGGGGGAATGAAGAAACCTGGTCCGAATTGC GGCCGCTATCTGGGTCTGTCTGTTGGCTGCC CGGTGACGGTATACGGTGATGTTCTAATAGTA CTCAATTGGTCTTGGATCGGAGTTCATGCTAC GGCTCCTCTGTTATATATTACACGGCTGACGG CTCCTTATTAATGTGTCCGTTGATGATGTCATT AATAATATGAATCTGATATTGTATAGGGGGTGT GTACTGCTGATTCCTCATGCGTCGTCGGAGAT CGAGAGGTGATGCATGCTGCGAACGGCGAGC GGCGGCGGCGGCGACAGGAGGTCGGCGGCG AGCTCCGGCGTGCTGGCTGGCATCCGGCGAT CGACGACGGCGACCGAGGGAGGGAGGAAGC |
| | TATAGCTAGGATGGTCATCATGTGATTAATTCA GTGATGTGTCCTAATTAAGTAGCTTCTTGCTTG CTAGCGGAGCTCCCTCACTCAATCCAAATCTG TAACCTTTTTCTTTCTTTCTGAAGTAAACCTTAA TAACCCAGCTATGTTCTCTTATGATGTTCTGAA CAAATACAACAAATTAGGCATACAGATTCAGAT ACAATAGCACTTGAGTTTGTATCTAGTCGTTTG TCAAGAACCAAGACACATTTCTTG |

### Example 2: Suppressing endogenous miRNA activity in dicots via target mimics

Soybean databases were Blastn searched for sequences containing a sequence similar to the motif TAGGGCAACTTNNNTCCTTTGGCA (SEQ ID NO. 30), where N is any nucleotide base. 6 soybean sequences having a similar motif were identified and are reported in Table 11. Sequence 51129502.f_I14_1 exactly matches part of sequence GM06MC32536_sk84d08 over its entire length. Sequence 52202528.f_f03_1 exactly matches part of sequence GM06MC09408-52318733 over its entire length.

**Table 11. Soybean target mimicry candidates**

| Sequence name | source | sequence |
|---|---|---|
| GM06MC32536_sk84d 08 | Soybean | AGAAACAAAATCCCTATAACACCCAA TCCTAGCTACAACTTCAAACCCTCTC TGAATTGCACCACCCCCATCATCCC AAACATGTAACACCCCTTTTGAAACT TTTTCTCATGAGCCACCCCCCCTTTG CCCCATGCCACCACTTCCTTAATCAA TTTGGCATGTGGGGGTGGTGAACAT GTTGTGGATATATGGTGTGTTTGGT GTGGCTATAATGGTATCTTCCTTATC TTTCCATGCTCCTTTTCTTTCAAGGC TAAGCGAAAACGCTCCTTCTTGAGG GGCGGTTTCTGTTGGAAAGGGCAAC TTCAATCCTTTGGCATTTTTCAACTC |
| | Hyseq_soybe an_mefi_v6.n t (SEQ ID NO.7) | |
| | | CTCCATCTCACCTCACACACTGTGAT TTGGGCTCTTGAGGGACATCTCAGA ATGGCAACACCCCTTTTTATGTGTAC CGTACTATTTTCTAGTTCTTTTTCTTG TACAACGAAATTTAGATGCAGTTTCT TAGATGTTGGTGTTGTTTTTTAATCA AAATTAGAGTTTTATCTTGATAGTAC CGGATTATCAAGGTAAAACTCTGATT CTTATCAGAAAATGGCACCAAAAATA CTTTAAGAACTGTATGCAAGTTTTTT GTTGTTTTTTTTTGGTAATTCTTGTTT GTGATGTGATGATTTCAACTGCAAAC TATGCCTATCAGTATGGTTACCATAG ACTATATGTTATCTAATAAATTTACAT TTGAATAGGCCTTTGGCCAAAATGTA TTATTGTTGTCGTTAATATGAATATG GTTAAATCAAAAAAAAAAAAAAAAAA AAAAAAAAAAAAAAAAAAAAAAAAA AAAAAAAAAAAAAAAAAAAAAAAAA AAAAAAAAAAAAAAAAAAAAATA |
| 51129502.f_I14_1 | Soybean | CCCATCATCCCAAACATGTAACACC CCTTTTGAAACTTTTTCTCATGAGCC ACCCCCCCTTTGCCCCATGCCACCA CTTCCTTAATCAATTTGGCATGTGGG GGTGGTGAACATGTTGTGGATATAT GGTGTGTTTGGTGTGGCTATAATGG TATCTTCCTTATCTTTCCATGCTCCTT TTCTTTCAAGGCTAAGCGAAAACGCT CCTTCTTGAGGGGCGGTTTCTGTTG GAAAGGGCAACTTCAATCCTTTGGC ATTTTTCAACTCCTCCATCTCACCTC ACACACTGTGATTTGGGCTCTTGAG GGACATCTCAGAATGGCAACACCCC TTTTTATGTGTACCGTACTATTTTCTA |
| | Hyseq_soybe an_EST.nt (SEQ ID NO.8) | |
| | | GTTCTTTTTCTTGTACAACGAAATTTA GATGCAGTTTC |
| GM06MC32394_sk51f 08 | soybean | CACGCGTCCGATTCATTCTCGAACC CTCTCCAAACTGCACCCCTTCTCGA ATAATAGCCCTCGCTGCCTCTGTGG CAGTGGAGGTGGTGGAGATGCATAT GGTGCATTTTTGTCGGTGTTTCCTTT TGGTTCAATCATGGTGGAGCATGAA TATTATGTAATAAGTTGAAAGATTGC TGGGAATGAACCGTTCTTTTTCTTAA GGGCGAATTCTCTTCAAAAGGGCAA CTTCCATCCTTTGGCATTTTCCAATT CCTCGAACTCACTTCACTTTGTGTGT GAATCAAGTTCTCAGGGGAACTTTG GCAATATATATGTAATATCCCTATAC TATGTACTATTAATTGTACTCTTTGTT ATCTTGTACTTTGTGTTTAAATTAGAA TTGCATTATATATATATATATATATAT ATATATATATATATATATATATGTACA CTATCAAGTATCAATATGTATGATAT GATGCTGCCTAATATATAGTATAATA GGCAGCCAATAAATGTTGGTTTTTAG AGAACTG |
| | Hyseq_soybe an-mefi-v6.n t (SEQ ID NO.9) | |
| GM06MC09408_52318 733 | Soybean | TGCTCCCCCTTCTCGAATAACCCTC GCTGCCCTCTGTGGTAGTGGAGGTG GTGGAGATTCATAGATGGTGCGTTT TTGTCAATGTTTCCTTTTGGTTCAAT CATGGTCGAGAAAGAATATGTAATAA GTTAAAAGATTGCTGGGAATGACGA ACCGTCCTTCTTAAGGGCGAATTCC CTCGAAAAGGGCAACTTCCATCCTTT GGCATTTTCCAATTAATTCCTCAAAC TCACTTCACTTTGTGTGTGAATCAAG TTCTCGAAGGAACTTTGGCAATATAG |
| | Hyseq_soybe an-mefi-v6.n t (SEQ ID NO.10) | |
| | | TATATAATATCCCTATACTATGTACTA TATACTGTACTCTTTGTTATCTTGTAC TTTGTGCTTCAATTAGAACTGCATTT TATATGTATCAGTGTATCACTATCAA CATATATGATATGATACTGCCTACTA TATATAGTAGGCAACCAATAAATGTT GGTTTTTAGACAAAAAAAAAAAAAAA AAGGGGCCGCTCTAAAGGATCCAAG TTTACGTACGCGGGCTGGCAAAGAA AAACTTTTT |
| | | |
| 53230190.f_b11_1 | Soybean | CTTAACCAATTTGGCATGTGGGGGT GGTGGACATGTTGTGGATGGTGTGT TTTGGTGTGGCTATAATGGTATCCTC CTTATCTTTCCATGCTCCTTTTCTTTC AAGGCTATGCTAAAACGCTCCTTCTT GAGGGCGGCTTCTCTTGGAAAGGG CAACTTCAATCCTTTGGCATTTTTCA ACTCCTCCACCTCACTTCACACACTG TGATTTGGGCTCTTGAGGGACCTCT CAGAATGGCAACACCCCTTTTATGT GTACCGTACTCTCTTCTAGTTCTTTT CTTGTACAACAAAATTTAGATGCAGT TTCTTACATGTTGGTGTTGCTTTCTA ATCAAAATTGGAGTTTTACCTTGGTG GCACCCAGATTATCGAGGTAAAACT CTGATTGTGATAGAAAAATGACACCA AAAACACT |
| | Hyseq_soybe an_EST.nt (SEQ ID NO.11) | |
| 52202528.f_f03_1 | Soybean | ATTGCTGGGAATGACGAACCGTCCT TCTTAAGGGCGAATTCCCTCGAAAA GGGCAACTTCCATCCTTTGGCATTTT CCAATTAATTCCTCAAACTCACTTCA CTTTGTGTGTGAATCAAGTTCTCGAA GGAACTTTGGCAATATAGTATATAAT |
| | Hyseq_soybe an_EST.nt (SEQ ID NO.12) | |
| | | ATCCCTATACTATGTACTATATACTG TACTCTTTGTTATCTTGTACTTTGTG CTTCAATTAGAACTGCATTTTATATG TATCAGTGTATCACTATCAACATATA TGATATGATACTGCCTACTATATATA GTAGGCAACCAATAAATGTTGGTTTT TAGACAAAAAAAAAAAAAAAAAAGGG GCCGCTCTAAAGGATCCAAGTTTAC GTACGCGGGCTGGCAAAGAAAAACT TTTT |

The 24 nt sequence having a motif similar to TAGGGCAACTTNNNTCCTTTGGCA (SEQ ID NO. 30) is underlined in each sequence.

Artificial miR159 target mimic.

To create a sequence that when expressed in soy will act as a target for endogenous miR159 miRNAs, sequence GM06MC32536_sk84d08 was modified as follows:
1. The sequence
   GTTTCTGTTGGAAAGGGCAACTTCAATCCTTTGGCATTTTTCAA (SEQ ID NO. 45) spanning nucleotides 287-330 of GM06MC32536_sk84d08 and including the motif similar to SEQ ID NO. 30 (underlined) was replaced with sequence
   GTTTCTGTTGGATAGAGCTCCCTCAATCAATCCAAATTTTTCAA(SEQ ID NO. 46) containing a miR159 target site.
2. The 91 nt poly(A) tail spanning nucleotides 760-850 in
   GM06MC32536_sk84d08 was deleted.

The final miR159 target sequence (Gm miR159-trap) is as follows with the miR159 target site underlined.

AGAAACAAAATCCCTATAACACCCAATCCTAGCTACAACTTCAAACCC
TCTCTGAATTGCACCACCCCCATCATCCCAAACATGTAACACCCCTTTTGAA
ACTTTTTCTCATGAGCCACCCCCCCTTTGCCCCATGCCACCACTTCCTTAAT
CAATTTGGCATGTGGGGGTGGTGAACATGTTGTGGATATATGGTGTGTTTGG
TGTGGCTATAATGGTATCTTCCTTATCTTTCCATGCTCCTTTTCTTTCAAGGC
TAAGCGAAAACGCTCCTTCTTGAGGGGCGGTTTCTGTTGGATAGAGCTCCC
TCAATCAATCCAAATTTTTCAACTCCTCCATCTCACCTCACACACTGTGATTT
GGGCTCTTGAGGGACATCTCAGAATGGCAACACCCCTTTTTATGTGTACCGT
ACTATTTTCTAGTTCTTTTTCTTGTACAACGAAATTTAGATGCAGTTTCTTAGA
TGTTGGTGTTGTTTTTTAATCAAAATTAGAGTTTTATCTTGATAGTACCGGATT
ATCAAGGTAAAACTCTGATTCTTATCAGAAAATGGCACCAAAAATACTTTAAG
AACTGTATGCAAGTTTTTTGTTGTTTTTTTTTGGTAATTCTTGTTTGTGATGTG
ATGATTTCAACTGCAAACTATGCCTATCAGTATGGTTACCATAGACTATATGT
TATCTAATAAATTTACATTTGAATAGGCCTTTGGCCAAAATGTATTATTGTTGT
CGTTAATATGAATATGGTTAAATC (SEQ ID NO. 47)

The miR159 target site is complementary to known soy miR159 family members in soy with a 3 nt bulge in the target sequence between bases 10 and 11 from the 5' end of the miRNA. miRNA sequences were obtained from miRbase (www.mirbase.org). RNA sequences are shown.

| | |
|---|---|
| Target site in Gm miR159-trap | 5'UAGAGCUCCCUCAAUCAAUCCAAA3' |
| (SEQ ID NO. 48) | |
| gma-MIR159a | 3'AUCUCGAGGGA AGUUAGGUUU5' |
| (SEQ ID NO. 49) | |
| gma-MIR159b | 3'ACCUCGAGGGA AGUGAGGUUA5' |
| (SEQ ID NO. 50) | |
| gma-MIR159c | 3'GCCUCGAGGGA AGUGAGGUUA5' |
| (SEQ ID NO. 51) | |

The Gm miR159-trap sequence was synthesized and cloned into a binary vector that contained the soy transformation selectable marker

AtAHASL2_A122T/S653N to create plasmide RTP4811-1qcz (Seq ID No.13). In RTP4811-1qcz. Gm miR159-trap was expressed under control of the Super promoter from BPS-LU and NOS terminator and AtAHASL2_A122T/S653N was expressed under control of the PcUbi4-2 promoter and AtAHAS-3'/UTR[ac321] terminator.

Partial sequence of RTP4811-1 from bases 4071 to 6215, including the elements p-SUPER (bases 4071-5182), c-Gm miR159-TRAP (bases 5191-5949), and t-NOS (bases 5963-6215) is presented as SEQ ID NO. 52.

Artificial miR159 target mimic control for soy (gma-miRandom1 target mimic).

To create a sequence that when expressed in soy will act as a control for Gm miR159-TRAP, sequence GM06MC32536_sk84d08 was modified as follows:
1. The sequence
   GTTTCTGTTGGAAAGGGCAACTTCAATCCTTTGGCATTTTTCAA (SEQ ID NO. 53) spanning nucleotides 287-330 of GM06MC32536_sk84d08 and including the motif similar to SEQ ID NO. 30 (underlined) was replaced with sequence
   GTTTCTGTTGGATGACTACGGTTCAAATACCTGTAATTTTTCAA (SEQ ID NO. 54) containing a miR159 target site.
3. The 91 nt poly(A) tail spanning nucleotides 760-850 in
   GM06MC32536_sk84d08 was deleted.

The sequence of the control (gma-miRandom1 target mimic) is identical to Gm miR159-trap except the target region has been modified so that it will not bind any known soy microRNA.

The final miRandom target sequence (gma-miRandom1 target mimic, SEQ ID NO. 14) is as follows with the miRandom target site underlined.

AGAAACAAAATCCCTATAACACCCAATCCTAGCTACAACTTCAAACCC
TCTCTGAATTGCACCACCCCCATCATCCCAAACATGTAACACCCCTTTTGAA
ACTTTTTCTCATGAGCCACCCCCCCTTTGCCCCATGCCACCACTTCCTTAAT
CAATTTGGCATGTGGGGGTGGTGAACATGTTGTGGATATATGGTGTGTTTGG
TGTGGCTATAATGGTATCTTCCTTATCTTTCCATGCTCCTTTTCTTTCAAGGC
TAAGCGAAAACGCTCCTTCTTGAGGGGCGGTTTCTGTTGGATGACTACGGTT

CAAATACCTGTAATTTTTCAACTCCTCCATCTCACCTCACACACTGTGATTTG
GGCTCTTGAGGGACATCTCAGAATGGCAACACCCCTTTTTATGTGTACCGTA
CTATTTTCTAGTTCTTTTTCTTGTACAACGAAATTTAGATGCAGTTTCTTAGAT
GTTGGTGTTGTTTTTTAATCAAAATTAGAGTTTTATCTTGATAGTACCGGATTA
TCAAGGTAAAACTCTGATTCTTATCAGAAAATGGCACCAAAAATACTTTAAGA
ACTGTATGCAAGTTTTTTGTTGTTTTTTTTTGGTAATTCTTGTTTGTGATGTGA
TGATTTCAACTGCAAACTATGCCTATCAGTATGGTTACCATAGACTATATGTT
ATCTAATAAATTTACATTTGAATAGGCCTTTGGCCAAAATGTATTATTGTTGTC
GTTAATATGAATATGGTTAAATC (SEQ ID NO. 14)

The gma-miRandom1 target mimic sequence was synthesized and cloned into a binary vector that contained the soy transformation selectable marker AtAHASL2_A122T/S653N to create plasmid RTP4808-1qcz (SEQ ID NO.15). In RTP4811-1qcz. gma-miRandom1 was expressed under control of the Super promoter from BPS-LU and NOS terminator and AtAHASL2_A122T/S653N was expressed under control of the PcUbi4-2 promoter and AtAHAS-3'/UTR[ac321] terminator.

Partial sequence of RTP4808-1 from bases 3593 to 5737, including the elements p-SUPER (bases 3593-4704), gma-miRandom1 target mimic (bases 4713-5471), and t-NOS (bases 5485-5737) is presented as SEQ ID NO. 55.

RTP4811-1 and RTP4808-1 soybean transgenic root lines were generated and RNA was extracted.

CO984960 is a predicted target of miR159 in soybean. Taqman probes were designed for CO984960 and the expression level was determined by quantitative RT-PCR in the RNA isolated from each root line.

The least squares mean for each population is reported in table 12 for gene CO984960. The p-values between the RTP4811-1 and RTP4808-1 are reported in table 13 demonstrating the higher expression levels of CO984960 in roots expressing Gm miR159-trap vs. gma-miRandom1 target mimic is significant.

**Table 12. Relative expression of CO984960 from transgenic soybean roots**

| | Relative CO984960 expression | |
|---|---|---|
| group | Least squares mean | Standard Error |
| RTP4808-1 | 5.33 | 1.35 |
| RTP4811-1 | 16.40 | 1.04 |

Table 13. Pvalue of relative expression of CO984960 from transgenic soybean roots

| | pvalue |
|---|---|
| RTP4808-1 | 1.4E-05 |

### Soy Target Mimic for Multiple microRNAs

To create a sequence that when expressed in soy will act as a target for multiple endogenous soy miRNAs, two GM06MC32536_sk84d08 sequences can be synthesized in tandem. The 91 nt poly(A) tail spanning nucleotides 760-850 in GM06MC32536_sk84d08 is deleted. The motif similar to SEQ ID NO. 30 can be replaced with a sequence that can potentially sequester any endogenous microRNA.

The following is an example of the stacked GM06MC32536_sk84d08 sequences with modifications to allow the transcript to act as a target mimic for miR159 and miR167. The final miR159 and miR167 target sequences (Gm miR159/miR167-trap, SEQ ID NO.16) is as follows with the miR159 and miR167 target sites underlined, respectively. The sequence includes the elements p-SUPER (bases 1-1112), gma-miR159/miR167 target mimic (bases 1113-2638), and t-NOS (bases 2639-2891).

GGATCCCTGAAAGCGACGTTGGATGTTAACATCTACAAATTGCCTTTTCTTAT
CGACCATGTACGTAAGCGCTTACGTTTTTGGTGGACCCTTGAGGAAACTGGT
AGCTGTTGTGGGCCTGTGGTCTCAAGATGGATCATTAATTTCCACCTTCACC
TACGATGGGGGGCATCGCACCGGTGAGTAATATTGTACGGCTAAGAGCGAA
TTTGGCCTGTAGGATCCCTGAAAGCGACGTTGGATGTTAACATCTACAAATT

GCCTTTTCTTATCGACCATGTACGTAAGCGCTTACGTTTTTGGTGGACCCTT
GAGGAAACTGGTAGCTGTTGTGGGCCTGTGGTCTCAAGATGGATCATTAATT
TCCACCTTCACCTACGATGGGGGCATCGCACCGGTGAGTAATATTGTACG
GCTAAGAGCGAATTTGGCCTGTAGGATCCCTGAAAGCGACGTTGGATGTTA
ACATCTACAAATTGCCTTTTCTTATCGACCATGTACGTAAGCGCTTACGTTTT
TGGTGGACCCTTGAGGAAACTGGTAGCTGTTGTGGGCCTGTGGTCTCAAGA
TGGATCATTAATTTCCACCTTCACCTACGATGGGGGCATCGCACCGGTGA
GTAATATTGTACGGCTAAGAGCGAATTTGGCCTGTAGGATCCGCGAGCTGG
TCAATCCCATTGCTTTTGAAGCAGCTCAACATTGATCTCTTTCTCGATCGAGG
GAGATTTTTCAAATCAGTGCGCAAGACGTGACGTAAGTATCCGAGTCAGTTT
TTATTTTTCTACTAATTTGGTCGTTTATTTCGGCGTGTAGGACATGGCAACCG
GGCCTGAATTTCGCGGGTATTCTGTTTCTATTCCAACTTTTTCTTGATCCGCA
GCCATTAACGACTTTTGAATAGATACGCTGACACGCCAAGCCTCGCTAGTCA
AAAGTGTACCAAACAACGCTTTACAGCAAGAACGGAATGCGCGTGACGCTC
GCGGTGACGCCATTTCGCCTTTTCAGAAATGGATAAATAGCCTTGCTTCCTA
TTATATCTTCCCAAATTACCAATACATTACACTAGCATCTGAATTTCATAACCA
ATCTCGATACACCAAATCGAAGAAACAAAATCCCTATAACACCCAATCCTAG
CTACAACTTCAAACCCTCTCTGAATTGCACCACCCCCATCATCCCAAACATG
TAACACCCCTTTTGAAACTTTTTCTCATGAGCCACCCCCCCTTTGCCCCATG
CCACCACTTCCTTAATCAATTTGGCATGTGGGGGTGGTGAACATGTTGTGGA
TATATGGTGTGTTTGGTGTGGCTATAATGGTATCTTCCTTATCTTTCCATGCT
CCTTTTCTTTCAAGGCTAAGCGAAAACGCTCCTTCTTGAGGGGCGGTTTCTG
TTGGATAGAGCTCCCTCAATCAATCCAAATTTTTCAACTCCTCCATCTCACCT
CACACACTGTGATTTGGGCTCTTGAGGGACATCTCAGAATGGCAACACCCC
TTTTTATGTGTACCGTACTATTTTCTAGTTCTTTTTCTTGTACAACGAAATTTA
GATGCAGTTTCTTAGATGTTGGTGTTGTTTTTTAATCAAAATTAGAGTTTTATC
TTGATAGTACCGGATTATCAAGGTAAAACTCTGATTCTTATCAGAAAATGGCA
CCAAAAATACTTTAAGAACTGTATGCAAGTTTTTTGTTGTTTTTTTTTGGTAAT
TCTTGTTTGTGATGTGATGATTTCAACTGCAAACTATGCCTATCAGTATGGTT
ACCATAGACTATATGTTATCTAATAAATTTACATTTGAATAGGCCTTTGGCCA
AAATGTATTATTGTTGTCGTTAATATGAATATGGTTAAATCGCGGCCGCAGAA
ACAAAATCCCTATAACACCCAATCCTAGCTACAACTTCAAACCCTCTCTGAAT
TGCACCACCCCCATCATCCCAAACATGTAACACCCCTTTTGAAACTTTTTCTC
ATGAGCCACCCCCCCTTTGCCCCATGCCACCACTTCCTTAATCAATTTGGCA

TGTGGGGGTGGTGAACATGTTGTGGATATATGGTGTGTTTGGTGTGGCTATA
ATGGTATCTTCCTTATCTTTCCATGCTCCTTTTCTTTCAAGGCTAAGCGAAAA
CGCTCCTTCTTGAGGGGCGGTTTCTGTTGGATAGATCATGCTCAAGGCAGC
TTCATTTTTCAACTCCTCCATCTCACCTCACACACTGTGATTTGGGCTCTTGA
GGGACATCTCAGAATGGCAACACCCCTTTTTATGTGTACCGTACTATTTTCTA
GTTCTTTTTCTTGTACAACGAAATTTAGATGCAGTTTCTTAGATGTTGGTGTT
GTTTTTTAATCAAAATTAGAGTTTTATCTTGATAGTACCGGATTATCAAGGTAA
AACTCTGATTCTTATCAGAAAATGGCACCAAAAATACTTTAAGAACTGTATGC
AAGTTTTTTGTTGTTTTTTTTTGGTAATTCTTGTTTGTGATGTGATGATTTCAA
CTGCAAACTATGCCTATCAGTATGGTTACCATAGACTATATGTTATCTAATAA
ATTTACATTTGAATAGGCCTTTGGCCAAAATGTATTATTGTTGTCGTTAATAT
GAATATGGTTAAATCGATCGTTCAAACATTTGGCAATAAAGTTTCTTAAGATT
GAATCCTGTTGCCGGTCTTGCGATGATTATCATATAATTTCTGTTGAATTACG
TTAAGCATGTAATAATTAACATGTAATGCATGACGTTATTTATGAGATGGGTT
TTTATGATTAGAGTCCCGCAATTATACATTTAATACGCGATAGAAAACAAAAT
ATAGCGCGCAAACTAGGATAAATTATCGCGCGCGGTGTCATCTATGTTACTA
GATC (SEQ ID NO. 56)

Target mimic for downregulation of multiple miRNAs:

To create a sequence that when expressed in a monocot will act as a target for multiple endogenous small regulating RNAs, two Gm miR166-trap sequences (Table 2) can be expressed in tandem as one transcript with each sequence containing a target site for a different small regulating RNA. In table 9, is a tandem construct with target sites for miR166 (first 24nt sequence underlined) and miR159 (second 24nt sequence underlined).

The Gm miR159-trap/Gm miR167-trap sequence was synthesized and cloned into a binary vector that contained the soy transformation selectable marker AtAHASL2_A122T/S653N to create plasmid RWT1000 (SEQ ID NO.17). In RWT1000. Gm miR159-trap/Gm miR167-trap was expressed under control of the Super promoter from BPS-LU and NOS terminator and AtAHASL2_A122T/S653N was expressed under control of the PcUbi4-2 promoter and AtAHAS-3'/UTR[ac321] terminator.

### Example 3: Suppressing endogenous miRNA activity via miRNA trap

The concept of this miRNA trap is to embed a modified miRNA binding site in the 3' untranslated region of transgene expression cassette. The modification is to insert a few (e.g. three) nucleotides between position 10 and 11 of the miRNA where cleavage of target mRNA by miRNA is normally taken place. Because of three extra nucleotide insertion at the cleavage site, miRNA would still bind to the modified site (or miRNA trap) but is incapable of cleaving target mRNA. Thus, miRNA trap serves as a 'sink' to sequester or suppress endogenous miRNA activity.

Construct RPR44 (SED ID NO. 18) carrying an expression cassette consist of dsRed reporter gene under the control of sugarcane bacilliform virus promoter and nopaline synthase (NOS) terminator. Between the stop codon of dsRed, TAG, and NOS terminator, a 24-nt DNA fragment (5' AAGGGGTGACCTAAGAACACCACC 3' (SEQ ID NO. 57)) complimentary to miR398a.

miRNA profiling with miRNA specific assays developed by Applied Biosystem on a range of wild-type maize tissues indicated that miR398a is highly expressed in embryo, weak in endosperm and not in leaf, root and tassel. Analysis of transgenic maize events transformed with RPR44 indicated significant reduction in dsRed fluorescence in embryo but not in endosperm, root and tassel indicated a reciprocal correlation of dsRed and miRNA398a expression, i.e. miR398a recognizes its binding site in the 3' untranslated region of dsRed expression cassette and cleaves dsRed mRNA in the embryo. Reduction of dsRed expression in leaf was unexpected, likely due to expression of other miR398a family members in the leaf that were not detected by miR398a assay.

Construct RTP3429-9qcz (SEQ ID NO.19) was identical to RPR44 except three nucleotides, CTA, was incorporated into 24-nt DNA fragment at miR398a cleavage site, i.e. 5' AAGGGGTGACCCTATAAGAACACC 3' (SEQ ID NO. 58) (miR398a trap). As a result, a bulge is formed between miR398a and miR398a trap causing dsRed transcripts uncleavable by miR398a.

RTP3429-9qcz was transformed into maize. dsRed expression from leaf of T0 transgenic events was analyzed. At least 20% of events had strong dsRed expression in leaf and rest of events had weak dsRed expression judged by fluorescence. This result suggested that miR398a binds to modified miR398a site without cleaving the transcript. Thus, miRNA trap approach is capable to suppress endogenous miRNA activity.

### Example 4: Suppressing endogenous miRNA activity by using an antisense transcript of the miRNA precursor

The method is to capture specific miRNAs and up-regulate the miRNA targets. By over expressing the complementary strand of an endogenous pre-miRNA, the effective concentration of the endogenous miRNA available to cleave its natural targets is reduced. The first possible mode of action for complementary transcript is to form double-stranded RNA with endogenous pre-miRNA and result in silencing of pre-miRNA. The second possible mode of action for complementary transcript is to serves as an additional cleavage target for mature miRNA. Since the complement is expressed at a high level it becomes the dominant target by the miRNA essentially sequestering it away from the endogenous targets. Both mode of actions result in the up-regulation of the endogenous mRNA targeted by miRNA.

### Construct design and construction

The genomic DNA fragments complementary to miRNA precursors were identified from *Arabidopsis* genome (wvvw.arabidopsis.org). Using Primer 3 software, a set of specific PCR primers were generated for PCR amplifying each DNA fragment. Through Gateway cloning strategy, PCR products were cloned into entry vectors first, then into destination or binary vectors. The complements of six known pre-miRNAs were selected for analysis (Table 14). Expression of pre-miRNA complement is under the control of parsley ubiquitin promoter.

**Table 14. Summary of constructs and miRNA precursors targeted by antisense transgene**

| RCB Construct | Transgene | Complement of: | Validated Targets of miRNA |
|---|---|---|---|
| RCB346 | AT_P_MIR11 | pre-miR393a | At1g12820 At3g26810 At3g23690 At 3g62980 At4g03190 |
| RCB383 | AT_P_MIR60 | pre-miR396b | At2g22840 At2g36400 At2g45480 At4g24150 At4g37740 At5g53660 |
| RCB478 | ATint1070848 | pre miR167b | At1 g30330 At5g37020 |
| RCB493 | ATint1445292 | pre-miR166 | At1g30490 At1g52150 At2g34710 At5g60690 At4g32880 |

In RCB346 (SEQ ID NO.20), the transgene AT_P_MIR11 was expressed under the control of the PcUbi4-2 promoter and the NOS terminator. The selectable marker for plant transformation, the BAR gene, was expressed under the control of the NOS promoter and the NOS terminator.

Partial sequence of RCB346 from bases 7039-9489 including elements p-PcUbi4-2 (bases 7039-7363), AT_P_MIR11 (bases 8083-9194), and t-NOS (bases 9237-9489) is presented as SEQ ID NO. 59.

In RCB371 (SEQ ID NO.21), the transgene AT_P_MIR44 was expressed under the control of the PcUbi4-2 promoter and the NOS terminator. The selectable marker for plant transformation, the BAR gene, was expressed under the control of the NOS promoter and the NOS terminator.

Partial sequence of RCB371 from bases 7039-8856 including elements p-PcUbi4-2 (bases 7039-7363), AT_P_MIR44 (bases 8083-8561), and t-NOS (bases 8604-8856) is presented as SEQ ID NO. 60.

In RCB383 (SEQ ID NO.22), the transgene AT_P_MIR60 was expressed under the control of the PcUbi4-2 promoter and the NOS terminator. The selectable marker for plant transformation, the BAR gene, was expressed under the control of the NOS promoter and the NOS terminator.

Partial sequence of RCB383 from bases 7039-9293 including elements p-PcUbi4-2 (bases 7039-7363), AT_P_MIR60 (bases 8083-8998), and t-NOS (bases 9041-9293) is presented as SEQ ID NO. 61.

In RCB388 (SEQ ID NO.23), the transgene AT_P_MIR68 was expressed under the control of the PcUbi4-2 promoter and the NOS terminator. The selectable marker for plant transformation, the BAR gene, was expressed under the control of the NOS promoter and the NOS terminator.

Partial sequence of RCB388 from bases 7039-9258 including elements p-PcUbi4-2 (bases 7039-7363), AT_P_MIR68 (bases 8083-8963), and t-NOS (bases 9006-9258) is presented as SEQ ID NO. 62.

In RCB478 (SEQ ID NO.24), the transgene ATint1070848 was expressed under the control of the PcUbi4-2 promoter and the NOS terminator. The selectable marker for plant transformation, the BAR gene, was expressed under the control of the NOS promoter and the NOS terminator.

Partial sequence of RCB478 from bases 7038-9409 including elements p-PcUbi4-2 (bases 7039-7362), ATint1070848 (bases 8082-9114), and t-NOS (bases 9157-9409) is presented as SEQ ID NO. 63.

In RCB493 (SEQ ID NO.25), the transgene ATint1445292 was expressed under the control of the PcUbi4-2 promoter and the NOS terminator. The selectable marker for plant transformation, the BAR gene, was expressed under the control of the NOS promoter and the NOS terminator.

Partial sequence of RCB93 from bases 7038-9228 including elements p-PcUbi4-2 (bases 7039-7362), ATint1445292 (bases 8082-8933), and t-NOS (bases 8976-9228) is presented as SEQ ID NO. 64.

### Event Development and Selection

*Arabidopsis thaliana* ecotype C24 was transformed with approximately 1 kb of the complement strand of an endogenous miRNA gene and six lines were recovered that expressed a complement to a known miRNA. T2 seeds for each of the selected events were sown to selection media. Seedlings were harvested 14 days after sowing (DAS). The seedlings were blotted dry and stored at -80°C. Total RNA was extracted using Trizol per manufacturer's protocol, (Invitrogen, Carlsbad, California ( http://www.invitrogen.com/site/us/en/home.html ). The RNA was quantified on a Nanodrop 1000 ( www.nanodrop.com). The quality of the RNA was confirmed by analysis on the Agilent 2100 Bioanalyser (www.agilent.com)

### Target Selection

Gene sequences for validated targets of known miRNAs in *A*. *thanliana* are available from the *Arabidopsis* MPSS website, ( http://mpss.udel.edu/at/). Analysis of seedling tissue expression was surmised using the expression data provided for each target.

### Endogenous miRNA Analysis

The average expression levels of six miRNAs in complementary transgenics and controls was determined. miR393a also showed a large percentage reduction but was not significant. Analysis of the expression levels of miR396b in the individual lines indicated a range of values while the relative expression levels in the control group was more consistent but reduced from the non-transgenic Col-O control, *(Table14).*

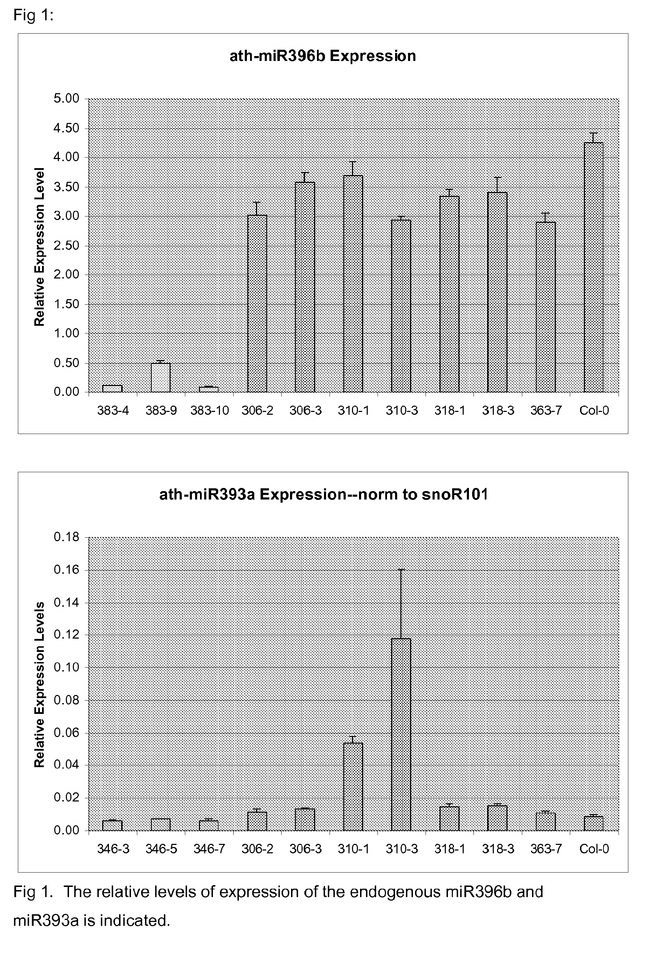

The reduction of the endogenous miRNA in each complement event was measured using the ABI method. The change was quantified using a custom designed RT primer for ath-miR396b and ath-miR393a from Applied Biosystems. Quantitative RT-PCR was performed and normalized to the expression of the endogenous snoR101 transcript. Only the expression level of miR396b in the RCB383 transgenic was significantly reduced from 9-46 fold or more than 90%, *(**Fig 1**, Table 15 and 16)..* The reduction of miR393a was not significant but showed some impact on a target gene. As shown in Figure 1, all three RCB383 events show a significant reduction in expression compared to the control group. The reduction of miR393a was less dramatic and not significant.

**Table 15. The average relative expression levels of the six miRNAs in are shown and compared with the levels in the control group and Columbia-O.**

| | | Average Relative Expression levels of miRNA | | |
|---|---|---|---|---|
| Construct | miRNA | Transgenic Events | Transgenic Controls | Col-0 |
| RCB346 | ath-miR393a | 0.006 | 0.034 | 0.008 |
| RCB371 | ath-miR169d | 0.187 | 0.175 | 1.783 |
| RCB383 | ath-miR396b | 0.234 | 3.266 | 4.252 |
| RCB388 | ath-miR156a | 5.899 | 5.108 | 10.138 |
| RCB478 | ath-miR167a | 29.749 | 36.406 | 54.435 |
| RCB493 | ath-miR166a | 8.015 | 6.197 | 8.821 |

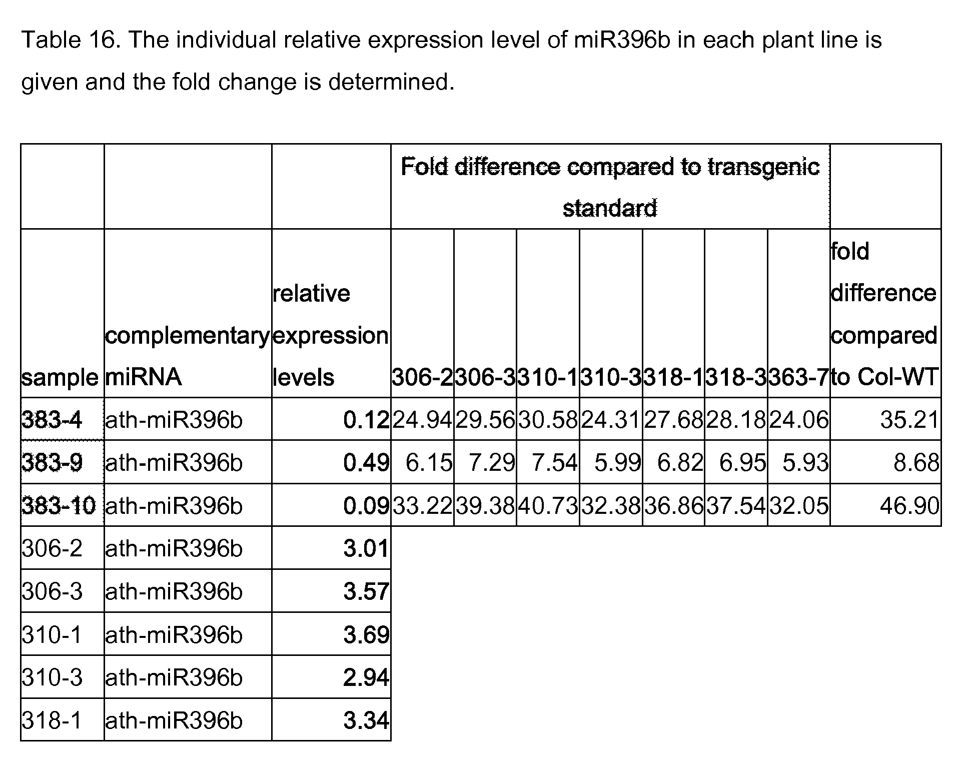

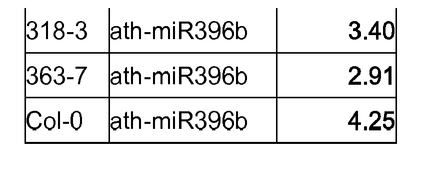

### Taqman Analysis

Taqman assays were devised for each target gene and validated on seedling RNA extracted from *Columbia* wildtype seedlings. At least two targets were selected for analysis for each complement, *(Table 14).* Selected targets were screened for a change in expression by TaqMan analysis when compared to the seven controls, (*Table17*).

**Table 17. The designation of the seven transgenic controls are provided. None expressed a complement to a known miRNA nor produced a phenotype either positive or negative in either whole plant assay.**

| Controls | | | | | | |
|---|---|---|---|---|---|---|
| RCB306-2 | RCB306-3 | RCB310-1 | RCB310-3 | RCB318-1 | RCB318-3 | RCB363-7 |

Three replicate of each assay were conducted simultaneously. The values were normalized against actin and an average expression of each in a plant was determined. The results showed little change in expression of the target genes compared to the control group except for two targets on each for RCB383 and RCB346, *(Table 18).* However at a confidence of 0.1 and 8 degrees of freedom a significant 3-fold increase in the expression of At2G22840 in the RCB383 transgenic and a 1.5-fold increase of At3G26810 in RCB346 transgenics was found. The increase expression of At2G22840 was correlated to the reduction in the endogenous miR396b. The 1.5-fold increase in At3G26810 was not supported by a significantly relevant reduction in the measured expression of miR393a, *(Table 19).*

**Table 18. The relative change in expression of the target gene in the three RCB383 transgenic events compared to the expression levels in the control group**

| | | | | | | | | |
|---|---|---|---|---|---|---|---|---|
| RCB383 | At2G228 40 | Ave Exp | Fold Increase in Express ion | | RCB346 | At3G62 980 | Ave Exp | Fold Increase in Expression |
| RCB383-10 | 0.094 | 0.130 | | | RCB346-7 | 0.378 | 0.305 | |
| RCB383-9 | 0.197 | | | | RCB346-3 | 0.250 | | |
| RCB383-4 | 0.100 | | 2.390 | | RCB346-5 | 0.287 | | 1.452 |
| Controls | | | | | Controls | | | |
| RCB306-2 | 0.144 | 0.054 | | | RCB306-2 | 0.298 | 0.210 | |
| RCB306-3 | 0.088 | | | | RCB306-3 | 0.249 | | |
| RCB310-1 | 0.035 | | | | RCB310-1 | 0.166 | | |
| RCB310-3 | 0.028 | | | | RCB310-3 | 0.130 | | |
| RCB318-1 | 0.018 | | | | RCB318-1 | 0.173 | | |
| RCB318-3 | 0.013 | | | | RCB318-3 | 0.192 | | |
| RCB363-7 | 0.056 | | | | RCB363-7 | 0.263 | | |
| | | | | | | | | |
| A. | | | | | B. | | | |

**Table 19. The table shows the average relative change in expression of the target genes in the transgenic events compared to the transgenic controls. The ratio of expression reflects the change in the expression of the target gene in the transgenic event. Values greater than 1 indicate an increase in expression while values less than one indicate a decrease in expression. Only changes with a probability less than 0.1 are considered significant when taken in conjunction to the ration of expression.**

| Target gene | Construct | miRNA | Probt | Ratio |
|---|---|---|---|---|
| At1G12820 | RCB346 | miR393a | 0.8891 | 0.981 |
| At3G23690 | RCB346 | miR393a | 0.9400 | 0.984 |
| At3G26810 | RCB346 | miR393a | 0.3888 | 0.912 |
| At3G62980 | RCB346 | miR393a | 0.0714 | 1.493 |
| At4G03190 | RCB346 | miR393a | 0.7460 | 1.045 |
| At2G22840 | RCB383 | miR396b | 0.0651 | 3.227 |
| At2G36400 | RCB383 | miR396b | 0.1898 | 1.386 |
| At4G37740 | RCB383 | miR396b | 0.4427 | 1.128 |
| At5G53660 | RCB383 | miR396b | 0.4125 | 0.673 |
| At1G30330 | RCB478 | miR4167b | 0.9046 | 1.015 |
| At5G37020 | RCB478 | miR4167b | 0.2881 | 1.476 |
| At2G34710 | RCB493 | miR166 | 0.4824 | 0.426 |
| At4G32880 | RCB493 | miR166 | 0.2918 | 1.647 |

### Analyze small RNA production in transgenic events by deep sequencing

RNA from RCB346 and RCB383 was deep sequenced for small RNAs. Both libraries were sequenced to similar depth with the RCB383 library being 97% that of the RCB346 library, *(Table 6).* The libraries were mapped to their respective genomes and transgenes. Since the transgene is the complement of a native gene a portion of the small RNA pool is expected to arise from the expression of the native gene. The portion of the small RNA pool expected from the native contribution was determined by cross mapping to the other construct. RCB346 was mapped onto RCB383 and 568 copies of pre-miR396b were found. RCB383 was mapped to RCB346 and 1218 copies of pre-miR393a were found. The overall contribution to the transgene small RNA pool was found to be quite small and primarily restricted to the miRNA and miRNAstar sequences. The deep sequencing results showed an approximate 75% reduction in the level of miR396b in RCB 383 when compared to miR396b levels in RCB346. The reduction was significant using a Chi Sq test to a p=5-E54. The overall numbers of miR393a in RCB346 and RCB383 were too small to provide an adequate assessment.

**Table. 20. Summary of small RNA analysis**

| Construct | Total small RNAs | Total small RNAs from the Endoge nous & Transgene | Total small RNAs from the Endogenous Gene | Native miR156 | miR396b | % Reducti on of miR396b | miR 393a | % Red uction of miR 393a |
|---|---|---|---|---|---|---|---|---|
| RCB383 | 13760979 | 8890 | 568 | 112617 | 130 | 74.7% | 9 | |
| RCB346 | 14268140 | 12912 | 1815 | 125030 | 514 | | 1 | 88.9 % |

### Summary

### Quantification of the Reduction of an Endogenous miRNAs

Analysis was conducted on all of the events determining the impact on the endogenous levels of the known miRNAs. RCB383 expresses the complement of miR396b and RCB346 expresses the complement of miR393a. A significant reduction in miR396b levels was found in the RCB383 transgenics. The ABI assay showed a significant 90% reduction while the deep sequencing results mirrored this with a 75% reduction. This indicates that the complement transcript served as a target sink resulting in the reduction in the miR396b levels. This reduction in the levels of miR396b resulted in an up regulation of the target gene At2g22840. A reduction in miR393a was found in RCB346 but the reduction was not significant. The deep sequencing results indicate that the natural levels of miR393a may be too small to make a meaningful determination of effectiveness.

The results here indicate that expressing the complementary strand of an endogenous miRNA serves as a means to reduce the expression levels of the endogenous miRNA. The sequestering of the miRNAs results in an up regulation of the expression of the natural targets of these miRNAs.

### Example 5: Suppressing endogenous small regulating RNA activity in dicots via target mimics.

The Arabidopsis IPS1 gene (Nature Genetics 2007. 39:1033-37.) that is reported to sequester the miRNA miR399 can be modified to down-regulate other endogenous small regulating RNAs other than miRNAs. Small regulating RNAs that can be down-regulated by target mimicry include nat-siRNAs, long siRNAs, and tasiRNAs.

### Arabidopsis miR399 target mimic

| Sequence name | source | sequence |
|---|---|---|
| IPS1 target mimic (SEQ ID NO: 26) | Arabidopsis | aagaaaaatggccatcccctagctaggtgaagaag aatgaaaacctctaatttatctagaggttattcatctttta ggggatggcctaaatacaaaatgaaaactctctaatt aagtggttttgtgttcatgtaaggaaagcgttttaagat atggagcaatgaagactgcagaaggctgattcaga ctgcgagttttgtttatctccctctagaaattgggcaact tctatcctttggcaagcttcggttcccctcggaatcagc agattatgtatctttaattttgtaatactctctctcttctctat gctttgtttttcttcattatgtttgggttgtacccactcccgc gcgttgtgtgttctttgtgtgaggaataaaaaatattc ggatttgagaactaaaactagagtagttttattgatatt cttgtttttcatttagtatctaataagtttggagaatagtc agaccagtgcatgtaaatttgcttccgattctctttatag tgaattcctc |

The 24 nt having a motif similar to TAGGGCAACTTNNNTCCTTTGGCA (SEQ ID NO. 30) is underlined.

### Artificial target mimic for nat-siRNA (SRO5-P5CDH)

To create a sequence that when expressed in soy will act as a target for endogenous nat-siRNA (SRO5-P5CDH), sequence IPS1 target mimic was modified as follows:
4. The sequence CTAGAAATTGGGCAACTTCTATCCTTTGGCAAGCTTCG (SEQ ID NO. 65) spanning nucleotides 211-248 of IPS1 and including the motif similar to SEQ ID NO. 30 (underlined) was replaced with sequence CTAGAAAGGGGACCCGAGAGGCTAGGCCGGGATAAGCTTCG(SEQ ID NO: 27) containing a nat-siRNA (SRO5-P5CDH) target site.

The nat-siRNA (SRO5-P5CDH) target site is complementary to known nat-siRNA (SRO5-P5CDH) in Arabidopsis with a 3 nt bulge in the target sequence between bases 10 and 11 from the 5' end of the miRNA.

| Target site in nat-siRNA (SRO5-P5CDH)-trap | |
|---|---|
| | 5'GGGGACCCGAGAGGCTAGGCCGGGATA |
| (SEQ ID NO. 66) | 3' |
| nat-siRNA (SRO5-P5CDH) | 3'CCCCTGGGCTCTCC CCGGCCCTAT 5' |
| (SEQ ID NO. 67) | |

### Artificial target mimic for the long siRNA, AtlsiRNA-1

To create a sequence that when expressed in soy will act as a target for endogenous AtlsiRNA-1, sequence IPS1 target mimic was modified as follows:
1. The sequence CTAGAAATTGGGCAACTTCTATCCTTTGGCAAGCTTCG (SEQ ID NO. 68) spanning nucleotides 211-248 of IPS1 and including the motif similar to SEQ ID NO. 30 (underlined) was replaced with sequence TAGAAAATCATCCAAAGTACAGTAACAGGCGATCTTCAGAACCAGAA GCATCTGTTCATCG AGCTTCG (SEQ ID NO:28) containing an AtlsiRNA-1 target site.

Target site in AtlsiRNA-1-trap

5'ATCATCCAAAGTACAGTAACAGGCGATCTTCAGAACCAGAAGCATCTGTTC
ATCG 3' (SEQ ID NO. 69)

AtlsiRNA-1

3’TAGTAGGTTTCATGTCATTGTCCGCTAGAAGTCTTGGTCTTCGTAGACAAG
TAGC 5’ (SEQ ID NO. 70)

### Artificial target mimic for D7 and D8 tasiRNAs from Arabidopsis and maize TAS3 gene

To create a sequence that when expressed in soy will act as a target for endogenous D7_and D8 tasiRNAs from the Arabidopsis TAS3 gene, sequence IPS1 target mimic was modified as follows:

### D7 tasiRNA target mimic

1. The sequence CTAGAAATTGGGCAACTTCTATCCTTTGGCAAGCTTCG (SEQ ID NO. 71) spanning nucleotides 211-248 of IPS1 and including the motif similar to SEQ ID NO. 30 (underlined) was replaced with sequence TAGAAA GGGGTCTTAGACTAAAGGTCAAGAA AGCTTCG (SEQ ID NO: 29) containing a D7 tasiRNA target site.

Target site in D7 tasiRNA-trap 5'GGGGTCTTAGACTAAGGTCAAGAA 3' (SEQ ID NO. 72)
D7 tasiRNA 3'CCCCAGAATCT TCCAGTTCTT 5' (SEQ ID NO. 73)

### D8 tasiRNA target mimic

1. The sequence CTAGAAATTGGGCAACTTCTATCCTTTGGCAAGCTTCG (SEQ ID NO. 74) spanning nucleotides 211-248 of IPS1 and including the motif similar to SEQ ID NO. 30 (underlined) was replaced with sequence TAGAAA GAGGCCTTACACTAAGGTCAAGAA AGCTTCG (SEQ ID NO. 75) containing a D8 tasiRNA target site.

| | |
|---|---|
| Target site in D8 tasiRNA-trap | 5'GAGGCCTTACACTAAGGTCAAGAA 3' (SEQ ID NO. 76) |
| D8 tasiRNA | 3'CTCCGGAATGT TCCAGTTCTT 5' (SEQ ID NO. 77) |

## Claims

1. A method for modulating, compared to a respective wild-type plant or part thereof, the expression of at least one target gene in a plant or part thereof, wherein the method comprises the following steps:
a) suppressing, compared to a respective wild-type plant or part thereof, the activity of at least one small regulating RNA (srRNA) molecule naturally targeting said at least one target gene by
b) introducing into said plant or part thereof at least one recombinant nucleic acid molecule not occurring in a respective wild-type plant or part thereof, wherein at least a part of said recombinant nucleic acid molecule is complementary to at least a part of at least one srRNA molecule or at least one precursor of said at least one srRNA molecule comprising said srRNA molecule regulating expression of said at least one target gene in said plant or part thereof.

2. The method according to claim 1 wherein the part of the recombinant nucleic acid molecule complementary to the precursor RNA molecule comprises at least 20 consecutive complementary base pairs of said precursor RNA molecule, said at least 20 consecutive complementary base pairs having at least 80% homology to the sequence of said precursor RNA molecule.

3. The method according to claim 1 or 2, wherein the part of the recombinant nucleic acid molecule complementary to the precursor RNA molecule comprises no sequences complementary to the srRNA sequence comprised in said precursor RNA molecule.

4. The method according to claim 1 or 2, wherein the part of the recombinant nucleic acid molecule complementary to the precursor RNA molecule comprises sequences which are complementary to the srRNA sequence comprised in said precursor RNA molecule.

5. The method according to claim 4 wherein the recombinant nucleic acid molecule comprising a sequence complementary to a srRNA sequence is selected from a nucleic acid molecule comprised in the group consisting of
a) a nucleic acid molecule represented by any of SEQ ID NO: 1, 2, 7, 8, 9,10,11,12 or 26 or
b) a nucleic acid molecule having at least 50 consecutive base pairs of a sequence described by any of SEQ ID NO: 1, 2, 7, 8, 9,10,11,12 or 26 or
c) a nucleic acid molecule having an identity of at least 70% over a sequence of at least 95 consecutive nucleic acid base pairs to a sequences described by any of SEQ ID NO: 1, 2, 7, 8, 9,10,11,12 or 26 or
d) a nucleic acid molecule hybridizing under medium stringent conditions with a nucleic acid molecule of at least 50 consecutive base pairs of a nucleic acid molecule described by any of SEQ ID NO: 1, 2, 7, 8, 9,10,11,12 or 26.

6. The method according to claim 5, wherein the part of the recombinant nucleic acid molecule complementary to the respective precursor RNA is represented by position 347 bp - 370 bp of SEQ ID NO: 1, position 234 bp - 257 bp of SEQ ID NO: 2, position 299 bp - 322 bp of SEQ ID NO: 7, position 233 bp - 256 bp of SEQ ID NO: 8, position 221 bp - 244 bp of SEQ ID NO: 9, position 184 bp - 207 bp of SEQ ID NO: 10, 149 bp - 172 bp of SEQ ID NO: 11, position 49 bp - 72 bp of SEQ ID NO: 12, and position 218 bp - 241 bp of SEQ ID NO: 26.

7. The method according to at least one of claims 4 to 6 wherein SEQ ID NO: 7, 8, 9, 10, 11, 12 and/or 26 is modulating the expression of a target gene in a dicotyledonous plant.

8. The method according to at least one of claims 4 to 6 wherein SEQ ID NO: 1 and/or 2 are modulating the expression of a target gene in a monocotyledonous plant.

9. The method according at least one of claims 4 to 8 wherein the sequence complementary to the respective precursor RNA molecule may be any of the sequences selected from the group of SEQ ID NO: 78 to 16344.

10. The method as claimed in claim 1 to 9 comprising the steps of
a) Identification of a srRNA sequence targeting the target gene
b) Optionally identification of the precursor RNA molecule comprising the respective srRNA sequence
c) Producing the recombinant molecule as defined in claims 1 - 9,
d) introducing the recombinant molecule into a plant or part thereof.

11. An isolated nucleic acid molecule comprising the sequence selected from the group of any one of
a) a nucleic acid molecule represented by any of SEQ ID NO: 1, 2, 7, 8, 9,10,11,12 or 26 or
b) a nucleic acid molecule having at least 50 consecutive base pairs of a sequence described by any of SEQ ID NO: 1, 2, 7, 8, 9,10,11,12 or 26 or
c) a nucleic acid molecule having an identity of at least 70%, over a sequence of at least 95 consecutive nucleic acid base pairs to a sequences described by any of SEQ ID NO: 1, 2, 7, 8, 9,10,11,12 or 26 or
d) a nucleic acid molecule hybridizing under medium stringency conditions with a nucleic acid molecule of at least 50 consecutive base pairs of a nucleic acid molecule described by any of SEQ ID NO: 1, 2, 7, 8, 9,10,11,12 or 26.

12. A nucleic acid construct for expression in plants comprising a nucleic acid molecule as defined in any of claims 1 - 11 functionally linked to a plant specific promoter.

13. A vector comprising a nucleic acid construct as defined in claim 12.

14. A microorganism able to transfer nucleic acids to a plant or part of a plant wherein said microorganism is comprising a recombinant nucleic acid construct as defined in claim 12 or a vector as defined in claim 13, wherein said recombinant nucleic acid molecule confers upon transfer of said recombinant nucleic acid construct to a plant or part of a plant a modulation of expression of a target gene in said plant or part of a plant compared to a respective plant or part of a plant not comprising said recombinant nucleic acid molecule.

15. A plant cell, a plant or part thereof comprising a recombinant nucleic acid construct as defined in claim 12 or a vector as defined in claim 13, wherein said recombinant nucleic acid molecule confers a modulation of expression of a target gene in said plant cell compared to a respective plant cell not comprising said recombinant nucleic acid molecule.
